(19) Europäisches Patentamt
European Patent Office
Office européen des brevets

(11) **EP 2 494 015 B1**

(12) **EUROPÄISCHE PATENTSCHRIFT**

(45) Veröffentlichungstag und Bekanntmachung des Hinweises auf die Patenterteilung:
**21.03.2018 Patentblatt 2018/12**

(51) Int Cl.:
***C11B 3/00*** *(2006.01)* ***C12N 9/16*** *(2006.01)*

(21) Anmeldenummer: **10774185.2**

(86) Internationale Anmeldenummer:
**PCT/EP2010/066234**

(22) Anmeldetag: **27.10.2010**

(87) Internationale Veröffentlichungsnummer:
**WO 2011/051322 (05.05.2011 Gazette 2011/18)**

(54) **KLONIERUNG, EXPRESSION UND VERWENDUNG EINER PHOSPHOLIPASE VON ASPERGILLUS FUMIGATUS**

CLONING, EXPRESSION AND USE OF A PHOSPHOLIPASE DERIVED FROM ASPERGILLUS FUMIGATUS

CLONAGE, EXPRESSION ET UTILISATION D'UNE PHOSPHOLIPASE D'APSERGILLUS FUMIGATUS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **28.10.2009 DE 102009051013**

(43) Veröffentlichungstag der Anmeldung:
**05.09.2012 Patentblatt 2012/36**

(73) Patentinhaber: **AB Enzymes GmbH 64293 Darmstadt (DE)**

(72) Erfinder:
• **NGUYEN, Quoc, Khanh 64385 Reichelsheim (DE)**
• **TITZE, Kornelia 64367 Mühltal (DE)**
• **SCHWARZ, Tatiana 64390 Erzhausen (DE)**
• **PALADINO, Silvia 64646 Heppenheim (DE)**
• **MARSCHNER, Volker 64404 Bickenbach (DE)**
• **LORENZ, Patrick 64653 Lorsch (DE)**

(74) Vertreter: **Hiebl, Inge Elisabeth Kraus & Weisert Patentanwälte PartGmbB Thomas-Wimmer-Ring 15 80539 München (DE)**

(56) Entgegenhaltungen:
**EP-A2- 0 513 709 WO-A1-98/31790**
**WO-A1-03/060112 WO-A1-2008/040466**
**WO-A2-03/012071**

• **ANNIKKA MUSTRANTA ET AL: "COMPARISON OF LIPASES AND PHOSPHOLIPASES IN THE HYDROLYSIS OF PHOSPHOLIPIDS", PROCESS BIOCHEMISTRY, XX, XX, Bd. 30, Nr. 5, 1. Januar 1995 (1995-01-01) , Seiten 393-401, XP000603907, DOI: DOI:10.1016/0032-9592(94)00030-L**
• **WINTER BRUNO H ET AL: "Application of phospholipases in the edible oil industry", FETT, Bd. 100, Nr. 4-5, Mai 1998 (1998-05), Seiten 152-156, XP002621175, ISSN: 0931-5985**
• **DATABASE EMBL [Online] 10 Februar 2010 'NOVEL PHOSPHOLIPASES AND USES THEREOF.' Retrieved from EBI, accession no. EM_PAT:DM383387 Database accession no. DM383387**

EP 2 494 015 B1

**Beschreibung**

Die Erfindung betrifft neue DNA-Sequenzen, die Polypeptide mit Phospholipaseaktivität im Wesentlichen ohne Lipaseaktivität kodieren. Die Erfindung betrifft ferner neue Polypeptide mit Phospholipaseaktivität im Wesentlichen ohne Lipaseaktivität. Bei diesen Polypeptiden handelt es sich um saure Phospholipasen mit niedrigem Molekulargewicht und hoher Thermostabilität bzw. hoher Temperaturbeständigkeit. Diese Polypeptide sind ferner über einen breiten pH-Bereich aktiv. Ferner betrifft die Erfindung auch die Verwendung dieser Phospholipasen zur Reduktion phosphorhaltiger Verbindungen, zum Beispiel bei der Herstellung von Speiseölen sowie die Verwendung dieser Phospholipasen als Backhilfsmittel, Tierfutterhilfsmittel, Hilfsmittel bei der Textilrohstoffverarbeitung, etc.

Rohe Pflanzenöle enthalten Begleitstoffe (z.B. freie Fettsäuren, Phospholipide, Schwermetalle, Farbstoffe,...) die bei der Lagerung durch die hydrolytische und oxidative Veränderung von Lipiden die Qualität und die Haltbarkeit des Öls beeinträchtigen und die weitere Verarbeitung erschweren. Deshalb ist eine Aufarbeitung (Raffination) nach der Gewinnung der rohen Pflanzenöle zur Beseitigung von unerwünschten Begleitstoffen notwendig. Bei der Herstellung von Speiseölen höchster Qualität, umfasst die Raffination die Schritte der Entschleimung, Bleichen und Desodorierung.

Der erste Schritt bei der Raffination von Ölen ist die Entschleimung. Die Entschleimung dient der Entfernung von Schleimstoffen, in erster Linie Phospholipiden, die eine negative geschmackliche Veränderung des Öles bewirken und bei den weiteren Verfahrenschritten der Ölraffination stören.

Da der Phosphorgehalt ein Maß für den Grad der Entschleimung ist, kann die Qualität des erhaltenen entschleimten Öls durch die Bestimmung des Rest-Phosphorgehalts beurteilt werden. Ein entschleimtes Öl mit einem Gehalt von Rest-Phosphor von weniger als 10ppm ist in weiteren Verarbeitungsschritten der Raffination erforderlich.

Phospholipide sind komplexe, phosphorhaltige Lipide. Phospholipide, wie Phosphatidylcholine oder Lecithin, bestehen aus einem Glyceringrundgerüst, das in den Positionen sn-1 und sn-2 mit Fettsäuren und in Position sn-3 mit einer estergebundenen Phosphatgruppe verestert ist. Die Phosphatgruppe ihrerseits kann wiederum z.B. mit einer primären Alkoholgruppe verestert sein. Natürliche Phospholipide enthalten an den Positionen sn-1 und sn-2 verschiedene Fettsäureketten, die im pflanzlichen Material überwiegend die mehrfach ungesättigten Acylketten sind. Es gibt zwei Arten von Phospholipiden, hydratisierbare und nicht-hydratisierbare. Zur Beseitigung von Phospholipiden werden unterschiedliche Methoden angewendet.

Die einfachste Methode ist die Wasserentschleimung. Bei diesem Verfahren können hydratisierbare Phospholipide mit Wasser ausgewaschen und aus dem Öl entfernt werden. Je nach Art und Qualität des Rohöls enthält das entschleimte Öl nach diesem Prozess noch 80-200 ppm Phosphor.

Bei der Acid-Entschleimungsmethode wird das Öl mit Säure behandelt. Die Säure bewirkt, dass nicht-hydratisierbare Phospholipide in hydratisierbare Phospholipide umgewandelt werden. Die hydratisierbaren Phospholipide werden ölunlöslich. Es bildet sich ölunlöslicher Schlamm, der aus dem Öl durch Zentrifugation oder Filtration entfernt wird. Nach diesem Verfahren beträgt der Restphosphorgehalt im entschleimten Öl ca. 25-100 ppm.

Die enzymatische Entschleimung bietet ein effizientes, kostengünstiges und umweltfreundliches Verfahren, um die Phospholipide schonend aus Speiseöl zu entfernen.

Phospholipasen sind Enzyme die Phospholipide spalten und werden entsprechend ihrer enzymatischen Spaltstellen am Phospholipid in Acylhydrolasen (Phospholipase A1, A2 und B) und Phosphodiesterasen (Phospholipase C und D) unterteilt. Die Phospholipase A1 (EC 3.1.1.32) hydrolysiert die Fettsäure an der sn-1 Position von Phospholipidmolekülen und die Phospholipase A2 (EC 3.1.1.4) spaltet spezifisch die sn-2 Esterbindung vom Phospholipid ab. Als Reaktionsprodukte entstehen das Lysophospholipid und die freie Fettsäure. Phospholipase B (EC 3.1.1.5) spaltet unspezifisch die Fettsäure an beiden sn-1 und sn-2 Positionen. Phospholipase C (EC 3.1.4.10) hydrolysiert die Phosphatesterbindung zwischen Glycerin und der Phosphatgruppe zu Phosphatmonoester und Diacylglycerol. Phospholipase D (EC 3.1.4.4) katalysiert die Hydrolyse der terminalen Phosphodiesterbindung, so dass Phosphatidinsäure und Cholin als Spaltprodukte entstehen.

Bekannt ist die Gewinnung der Phospholipase A2 aus Rinder- und Schweinepankreas, aus dem Gift der Honigbiene oder verschiedener Schlangenarten. Phospholipase kann auch aus Mikroorganismen, z.B. aus Bakterien und Pilzen gewonnen werden und durch rekombinante Techniken mit ausreichender Ausbeute produziert werden.

In dem Patent EP 0 513 709 wird erstmals ein wirksames enzymatisches Entschleimungsverfahren vorgestellt. Das neue Verfahren benutzt erstmals das Enzym Phospholipase A2, das die Fettsäure an der sn-2 Position von Phospholipiden abspaltet. Das Verfahren wurde an Soja- und Rapsöl mit einem Phosphorgehalt von 72 bis 110 ppm getestet. Der Reaktionsansatz enthielt bis 5 Gew.-% (w/w) Wasser, bezogen auf das Öl, und wurde bei pH 5,0 bis 5,5 bei 40°C bzw. 60°C bis zu 5 Stunden inkubiert. Das entstandene Lysophospholipid kann durch Zentrifugation aus dem Öl entfernt werden. Das entschleimte Öl weist danach ein Restphosphorgehalt von weniger als 5ppm auf.

Bei der Entschleimung wird dem Öl eine definierte Wassermenge zugemischt. Dann werden die enzymhaltige Wasserphase und die Ölphase miteinander vermischt, sodass das Enzym einwirken kann. Dabei sollte die Wassermenge möglichst klein gehalten werden. Die Verwendung großer Mengen an Wasser bedeutet einen erhöhten Energieaufwand und erhöhte Entsorgungskosten. Deshalb ist ein Prozess der enzymatischen Entschleimung mit geringem Wasseranteil (2%) schon in dieser Hinsicht vorteilhaft.

In US2007/134777 wird festgestellt, dass die enzymatische Entschleimung von Pflanzenöl mit Phospholipase A1 bei einem pH-Wert zwischen 4,0 und 5,0 durchgeführt wird. Der optimale pH-Wert für diese Reaktion liegt zwischen 4,5 und 5,0. In diesem pH-Bereich können sich die freigesetzten Calcium und/oder Magnesium-Ionen mit weiteren Chemikalien (Anionen) des Reaktionspuffers zu schwerlöslichen Salzen verbinden, die sich an der Oberfläche der Reaktoren ablagern und das Gerät dadurch verschmutzen. Die Beseitigung solcher Verschmutzungen und die Reinigung des Geräts sind aufwendig. Um eine Verschmutzung des Geräteinnenraums zu reduzieren, wird die Reaktion vorzugsweise bei einem pH-Wert von ca. 4 durchgeführt. Allerdings wird das Enzym weniger aktiv oder funktionell inaktiv, wenn der pH-Wert der Reaktion weiter absinkt.

In der EP 0 904 357 wird beschrieben, dass in *Aspergillus niger* eine Phospholipase A zur Entschleimung von Speiseöl gefunden wurde.

In WO2008/040466 wird beschieben, dass in *Aspergillus fumigatus* eine Phospholipase mit einem Molekulargewicht von 65kDa zur Entschleimung von Speiseöl mit 5% Wassergehalt bis zu Temperaturen von 65°C eingesetzt werden kann.

EP 1 788 080 beschreibt die Verwendung von Phospholipase C aus *Bacillus cereus* in der Entschleimung von Öl in 6 Stunden bei 60°C mit einem Wasseranteil von 15% bezogen auf das Öl. Der Restphosphorgehalt beträgt danach weniger als 5 ppm.

In WO2008/094847 wird beschrieben, dass die Reaktionszeit der Ölentschleimung unter dem Zusammenwirken der Phospholipase A1 bzw. A2 mit der Phospholipase C bis auf 30 min verkürzt werden kann.

Annikka Mustranta, Pirkko Forssell & Kaisa Poutanen: "Comparison of Lipases and Phospholipases in the Hydrolysis of Phospholipids", Process Biochemistry, Bd. 30, Nr. 5, 1. Januar 1995 (1995-01-01), Seiten 393-401, beschreibt den Vergleich von zwei handelsüblichen Pilzlipasepräparaten aus *Aspergillus niger und Penicillium cyclopium* von zwei Pilzphospholipasen aus *A. niger* ($A_1$ und $A_2$) und einer Phospholipase aus Schweinepancreas ($A_2$, Lecitase) bezüglich der Hydrolyse von Sojabohnenphospholipiden.

WO 98/31790 beschreibt ein Protein mit Phospholipaseaktivität, das dadurch gekennzeichnet ist, dass es die reife Sequenz der Aspergillus-Lysophospholipase oder eine davon abgeleitete Sequenz besitzt und an mindestens einer Stelle gespalten sein kann, wobei im Falle einer Spaltung die Spaltstücke entweder über mindestens eine unter reduzierenden Bedingungen spaltbare Verbindung verknüpft sind oder von den unverknüpften Spaltstücken mindestens eines Phospholipaseaktivität besitzt.

Phospholipasen werden ferner in der Lebens- und Futtermittelindustrie vielfältig eingesetzt, z.B. bei der Teigherstellung, bei der Herstellung von Backwaren, zur Erhöhung der Ausbeute bei der Käseherstellung etc. Daher werden Phospholipasen benötigt, die sich technologisch vielseitig einsetzen lassen.

In der Biotechnologie werden Phospholipasen als Biokatalysatoren für die Gewinnung von Phospholipiden verwendet. Phospholipide sind polare Lipide und wirken auf Grund lipophiler und hydrophiler Strukturmerkmale als Emulgatoren. Als Beispiel dient die Anwendung der Phospholipasen bei der Herstellung von modifizierten Lecithinen, als Nahrungsmittelemulsionen bei der Herstellung von Soßen, Majonäse und Salatdressing, bei der Herstellung von Instantpulvern, beispielsweise Milch-, Kakao- und Kaffeepulvern, als Fließverbesserer bei der Schokoladeherstellung und als Nahrungsergänzungsmittel. In der pharmazeutischen und kosmetischen Industrie werden die Phospholipide und Lysophospholipide zur Herstellung von Cremes, Lotionen, Gels und Liposomen-Formulierungen eingesetzt.

Lecithin wird auch zur Herstellung von Lacken, Farben, Magnetbändern, Spezialpapieren, Leder und Textilien benötigt. Phospholipasen werden auch in der Textilindustrie beim "Bioscouring" zur Reinigung der Pflanzenfaser vor den weiteren Bearbeitungsschritten wie z.B. dem Einfärben eingesetzt. Hierbei kann auch ein Gemisch aus Phospholipase zusammen mit anderen Enzymen zur Anwendung kommen. Die anderen Enzyme können aus der Gruppe der Cellulasen, Hemicellulasen, Pektinasen, Proteasen, und Oxidoreduktasen ausgewählt sein.

Es besteht daher im Stand der Technik ein konstanter Bedarf nach Phospholipasen mit möglichst breitem bzw. möglichst optimiertem Einsatzgebiet.

Der vorliegenden Erfindung liegt somit die Aufgabe zugrunde, Proteine bzw. Polypeptide mit verbesserten Phospholipaseeigenschaften bereitzustellen. Die neuen Phospholipasen sollen insbesondere keine in technologischen Prozessen relevante Lipaseaktivität aufweisen. Insbesondere sollen die Proteine mit Phospholipaseaktivität über einen weiten pH-Bereich aktiv sein bzw. eine hohe Temperaturbeständigkeit besitzen.

Ferner sollen die Proteine mit Phospholipaseaktivität einfach, kostengünstig und wirtschaftlich zu produzieren sein. Weiterhin sollen erfindungsgemäß Expressionskonstrukte bereitgestellt werden, die sich zur Produktion der Proteine mit Phospholipaseaktivität eignen.

Die vorstehenden Aufgaben werden gelöst durch ein Nukleinsäuremolekül mit einer DNA-Sequenz, die ein Polypeptid mit Phospholipaseaktivität im Wesentlichen ohne Lipaseaktivität, photomerisch bestimmt durch Hydrolyse von p-Nitrophenylpalmitat, kodiert, dadurch gekennzeichnet, dass die DNA-Sequenz ausgewählt ist aus

a) DNA-Sequenzen, die eine Nukleotidsequenz gemäß SEQ ID NO: 1 umfassen,

b) DNA-Sequenzen, die die kodierende Sequenz gemäß SEQ ID NO: 1 umfassen,

c) DNA-Sequenzen, die die Proteinsequenz gemäß SEQ ID NO: 2 kodieren,

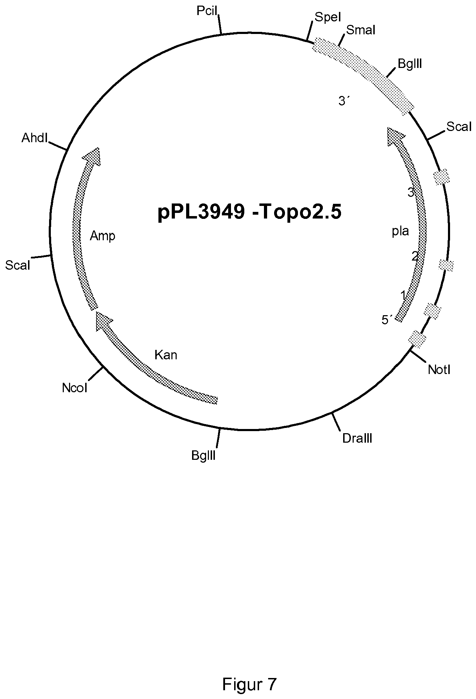

d) DNA-Sequenzen, die von dem Plasmid pPL3940-Topo2.5 mit der Restriktionskarte gemäß Figur 7 und hinterlegt unter der Hinterlegungsnummer DSM 22741 kodiert werden,

e) DNA-Sequenzen, die aufgrund der Degeneriertheit des genetischen Codes mit den DNA-Sequenzen gemäß a), b), c) oder d) verwandt sind, und

f) komplementären Strängen zu den Sequenzen gemäß a) bis e),

wobei die DNA-Sequenz bevorzugt aus *Aspergillus* und noch bevorzugter aus *Aspergillus fumigatus* abgeleitet ist, sowie ein Nukleinsäuremolekül, umfassend ein Analogon einer der Sequenzen gemäß Anspruch 1, dadurch gekennzeichnet, dass die Sequenz ein Polypeptid mit Phospholipaseaktivität im Wesentlichen ohne Lipaseaktivität, photomerisch bestimmt durch Hydrolyse von p-Nitrophenylpalmitat, kodiert und

a) mindestens 88% Identität zu einer der Sequenzen gemäß b), c), e) und f) aufweist, wobei die Sequenz erhältlich ist durch Substitution, Deletion, Insertion, Addition oder Mutation einer oder mehrerer Nukleinsäuren der DNA-Sequenz gemäß SEQ ID NO: 1 oder

b) ein komplementärer Strang zu den Sequenzen gemäß a) ist, wobei die Sequenz erhältlich ist durch Substitution, Deletion, Insertion, Addition oder Mutation einer oder mehrerer Nukleinsäuren der DNA-Sequenz gemäß SEQ ID NO: 1.

Die Erfindung betrifft ferner ein Polypeptid mit Phospholipaseaktivität im Wesentlichen ohne Lipaseaktivität, ausgewählt aus

a) einem Polypeptid, das von dem kodierenden Teil einer DNA-Sequenz nach einem der Ansprüche 1 bis 2 kodiert wird,

b) einem Polypeptid mit einer Sequenz, die mindestens 83% Identität zu den Aminosäuren 1 bis 299 von SEQ ID NO: 2 aufweist,

c) einem Polypeptid, das von einer Nukleinsäuresequenz kodiert wird, die unter stringenten Bedingungen hybridisiert mit

(i) den Nukleotiden 55 bis 1106 von SEQ ID NO: 1,

(ii) der in den Nukleotiden 55 bis 1106 von SEQ ID NO: 1 enthaltenen cDNA-Sequenz, oder

(iii) einem Komplementärstrang von (i) oder (ii).

Weiterhin betrifft die Erfindung Expressionskonstrukte bzw. Wirte, die in der Lage sind, die erfindungsgemäßen Polypeptide mit Phospholipaseaktivität zu exprimieren. Ferner betrifft die Erfindung auch die entsprechenden Expressionsplasmide und Vektoren. Weiterhin betrifft die Erfindung Verfahren zur Entschleimung von pflanzlichem Öl unter Verwendung der erfindungsgemäßen Polypeptide sowie die Verwendung der erfindungsgemäßen Polypeptide für Anwendungen im Bereich der Lebensmitteltechnologie, insbesondere zur Herstellung von Teig, Backwaren oder Milchprodukten, bzw. in der Tierernährung und bei der Bearbeitung von Textilrohstoffen, dem sogenannten Scouring oder Bioscouring.

Gemäß einer weiteren Ausführungsform betrifft die Erfindung ein Polypeptid mit Phospholipaseaktivität im Wesentlichen ohne Lipaseaktivität wie vorstehend definiert, das dadurch gekennzeichnet ist, dass es ein Molekulargewicht im Bereich von 28 bis 30 kDa und bevorzugt von ca. 28,6 kDa besitzt, mindestens eine der zwei Fettsäuren aus Lecithin hydrolysieren kann, im Wesentlichen keine Lipaseaktivität aufweist, eine hohe Temperaturbeständigkeit besitzt und aus einem Organismus der Gattung *Aspergillus* isolierbar ist.

Dabei ist unter hoher Temperaturbeständigkeit zu verstehen, dass das Enzym nach 6 h bei einer Temperatur von 60°C unter den Bedingungen bei der Ölentschleimung mit geringem Wasseranteil von 2% seine Aktivität in industriell verwertbarem Umfang beibehält. Das Enzym behält seine Aktivität in industriell verwertbarem Umfang bei, indem es zu Ölen mit technologisch praktisch unbedeutenden Restphosphorgehalten führt. Bevorzugt beträgt hierbei der Restphosphorgehalt im enzymatisch entschleimten Öl weniger als 10 ppm, noch bevorzugter weniger als 5 ppm.

Überraschenderweise wurde gefunden, dass eine DNA-Sequenz, die ein Polypeptid mit Phospholipaseaktivität im Wesentlichen ohne Lipaseaktivität kodiert, das ein niedriges Molekulargewicht sowie eine hohe Temperaturbeständigkeit besitzt, aus einem Stamm der Gattung *Aspergillus fumigatus* isoliert werden kann. Bei dieser Phospholipase handelt es sich um eine saure, von einem filamentösen Pilz abstammende Phospholipase mit einem berechneten Molekulargewicht von ca. 28,6 kDa, die in der Lage ist, mindestens eine der zwei Fettsäuren aus Lecithin zu hydrolysieren.

Im Gegensatz zu den Polypeptiden mit Phospholipaseaktivität, die aus dem Stand der Technik bekannt sind, verfügen die erfindungsgemäßen Phospholipasen über eine hohe Temperaturbeständigkeit (bei 60°C) und können dadurch vorteilhafterweise auch in Prozessen der enzymatischen Entschleimung mit geringem Wasseranteil von 2% (bezogen auf Öl) und bei einem pH-Wert von 4,0 eingesetzt werden. Dies ist vor allem ökonomisch interessant, da somit bei den Entschleimungsprozessen nicht zuerst die Temperatur des Öls erniedrigt werden muss, um die enzymatische Entschleimung ohne Inaktivierung des Enzyms zu ermöglichen und anschließend die Temperatur des Öls erhöht werden muss, um die Viskosität des Öls für den Zentrifugationsschritt zur Trennung der Öl- und Wasserphasen zu erniedrigen. Auch für andere Anwendungen im Bereich der Lebensmitteltechnologie und Tierernährung bzw. in der Textilverarbeitung ist die Temperaturbeständigkeit der erfindungsgemäßen Polypeptide mit Phospholipaseaktivität vorteilhaft.

Phospholipasen aus dem Stand der Technik sind vom Umfang der Erfindung ausgenommen.

Es ist deshalb sehr vorteilhaft, dass die erfindungsgemäßen von *A. fumigatus* abgeleiteten Phospholipasen in einem weiten pH-Bereich von 3 bis 5 aktiv sind bzw. in diesem Bereich ein sehr breites Aktivitätsoptimum zeigen. Die erfindungsgemäßen Phospholipasen besitzen somit nicht nur den Vorteil, dass sie im Wesentlichen keine Lipaseaktivität haben. Sie entfalten zusätzlich ihre Enzymaktivität über einen breiten pH-Bereich und sind somit über einen breiten pH-Bereich einsetzbar.

Enzyme mit Phospholipaseaktivität (Phospholipase A, B, C oder D) aus *Aspergillus fumigatus* wurden bis jetzt in verschiedenen Publikationen (Birch et al., Comparison of extracellular phospholipase activities in clinical and environmental Aspergillus fumigatus isolates, 2004, Med Mycol 42(1): 81-86; Rementeria et al., Genes and molecules involved in Aspergillus fumigatus virulence, 2005, Rev Iberoam Micol 22(1): 1-23) erwähnt, aber nicht genau charakterisiert. Aus der *Aspergillus fumigatus* Genom Sequenz (Nierman et al., Genomic sequence of the pathogenic and allergenic filamentous fungus Aspergillus fumigatus, 2005, Nature, 438(7071): 1151-6) wurden unter der Bezeichnung "conceptual translation" mehrere hypothetische Phospholipase- (A, B, C, D) und Lysophospholipasegene abgeleitet. So wurden z.B. eine hypothetische Phospholipase A mit 241 Aminosäuren und drei Lysophospholipasen Plb1, Plb2 und Plb3 mit hohem Molekulargewicht dargestellt. Weiterhin zeigt die Datenbank eine kleine hypothetische extrazelluläre Lipase mit 299 Aminosäuren und einem berechneten Molekulargewicht von ca. 28,5 kDa (GenBank EAL86100) und mehrere Lipasen, die zwischen 409 bis 587 Aminosäuren aufweisen.

Shen et al. (Characterisation and expression of phospholipase B from the opportunistic fungus Aspergillus fumigatus, 2004, FEMS Microbol Lett 239(1): 87-93), gelang die Klonierung und Charakterisierung von 3 Phospholipase B Genen aus *Aspergillus fumigatus.* Die sekretierten Proteine AfPL1 mit 633 Aminosäuren und AfPL3 mit 630 Aminosäuren haben ein Molekulargewicht von ca. 68 kDa. Das Protein AfPL2 ist ein zytosolisches Protein mit 588 Aminosäuren und besitzt ein Molekulargewicht von ca. 63 kDa.

Aus *Aspergillus fumigatus* RH3949 wurden ferner eine Phosphopholipase mit 633 Aminosäuren (WO2008/040466) und eine Lysophospholipase mit 611 Aminosäuren (WO2008/040465) isoliert.

Weiterhin wurde eine saure Phospholipase (pl 4,1) mit kleinem Molekulargewicht im Bereich von 28 bis 30 kDa im Genom von *Aspergillus fumigatus* RH3949 gefunden.

Bei der Proteinsequenzierung dieser "kleinen" Phospholipase zeigten die ersten 16 Aminosäuren am N-Terminus eine 93% Identität zur N-terminalen Sequenz der hypothetischen extrazellulären Lipase mit 299 Aminosäuren (GenBank EAL86100) aus *Aspergillus fumigatus* Af293. Die Identität der reifen (AA 30-299) Sequenz der "kleinen Phospholipase" aus RH3949 mit der Sequenz der hypothetischen extrazellulären Lipase beträgt hingegen nur rund 82%. Dies war insbesondere überraschend, da an vergleichbarer Stelle im Genom eines weiteren *Aspergillus fumigatus* Stammes (A1163) eine Sequenz mit erheblich höherer Identität zur hypothetischen Lipase aus Af293 gefunden worden war (Genbank EDP1054) (Fedorova et al., Genomic Islands in the Pathogenic Filamentous Fungus Aspergillus fumigatus , 2008, PloS Genet.4. e1000046) .

Im Gegensatz dazu besteht keine Übereinstimmung zwischen zwei N-terminalen Phospholipasesequenzen aus *Aspergillus fumigatus* Af293 (Phospholipase A mit 241 Aminosäuren, GenBank EAL85761) und *Aspergillus fumigatus* RH3949 (Phospholipase mit pl 4,1).

Somit unterscheidet sich die erfindungsgemäße Phospholipase sowohl von bekannten Phospholipasen aus *Aspergillus fumigatus* als auch von als Lipasen annotierten Sequenzen sehr nahe verwandter *Aspergillus fumigatus* Stämme wie Af293.

Aus den DNA-Sequenzdaten (GenBank AAHF01000011) für die hypothetische extrazelluläre Lipase (GenBank EAL86100) wurden erfindungsgemäß verschiedene Oligonukleotidprimer abgeleitet und synthetisiert.

Überraschenderweise wurde gefunden, dass mit Primern, die von der Genom-Sequenz des Stammes Af293 abgeleitet sind, die erfindungsgemäße DNA-Sequenz aus dem Aspergillus-Stamm RH3949 isoliert werden konnte (vgl. Bsp. 4). Dies war nicht zu erwarten gewesen, da sich der zur Amplifizierung genutzte Stamm RH3949 (ein Umweltisolat) von Af293 (klinisches Isolat) phänotypisch und damit höchstwahrscheinlich auch genotypisch (Sequenz) unterscheidet. So war es denn auch mit anderen Primerpaaren mit zu N2-3948 und Apal-3949 benachbarten Bindungsstellen auf der genomischen DNA von Stamm Af293 nicht möglich gewesen das Phospholipasegen aus RH3949 zu amplifizieren.

Die Amplifikation des Genes erfolgte mit Hilfe der Polymerasenkettenreaktion (PCR) aus genomischer DNA von *Aspergillus fumigatus* RH3949.

Die Temperaturbeständigkeit und das breite pH-Optimum der erfindungsgemäßen Phospholipase waren überraschend und aufgrund der im Stand der Technik beschriebenen Phospholipasen nicht zu erwarten. Zu keiner der im Stand der Technik beschriebenen natürlich vorkommenden Phospholipasen aus filamentösen Pilzen sind diese Eigenschaften beschrieben oder auch nur nahegelegt.

Die erfindungsgemäße Phospholipasesequenz gemäß SEQ ID NO: 2 wurde mit Phospholipasesequenzen aus dem Stand der Technik verglichen. Eine partielle Übereinstimmung auf der Ebene der Aminosäuresequenz wurde zu bekannten Aminosäuresequenzen aus anderen *Aspergillus*-Stämmen gefunden, d.h. die Übereinstimmung betrug 60% mit einer Lipase aus *Aspergillus tubingensis* (WO98/45453) oder mit einer Lysophospholipase aus *Aspergillus foetidus* (EP0808903), 59% mit einer *Aspergillus niger* Phospholipase (WO03/097825, WO98/31790).

Die Sequenz SEQ ID NO: 2 zeigt die höchste Identität von 82% mit einer hypothetischen Sequenz für extrazelluläre Lipase (Genbank EAL86100) aus *Aspergillus fumigatus* Af293 (Nierman et al., Genomic sequence of the pathogenic and allergenic filamentous fungus Aspergillus fumigatus, 2005, Nature, 438(7071): 1151-6).

Überraschenderweise besitzt die erfindungsgemäße, aus *Aspergillus fumigatus* RH3949 isolierte Phospholipase unter den Bedingungen der enzymatischen Entschleimung von Speiseöl keine für diesen Prozess relevante Lipaseaktivität. Ferner besitzt dieses Enzym im Gegensatz zu bisher bekannten Phospholipasen aus anderen *Aspergillus*-Arten ein außergewöhnlich breites pH-Optimum und eine hohe Temperaturbeständigkeit. Das erfindungsgemäße Enzym kann so in einem Verfahren zur enzymatischen Entschleimung von Speiseölen vorteilhaft eingesetzt werden, da sie im Öl keine oder lediglich nicht nennenswerte Anteile an Triglyceridbindungen hydrolysiert.

Die Erfindung betrifft ferner auch Polypeptide mit Phospholipaseaktivität im Wesentlichen ohne Lipaseaktivität mit einer Sequenz, die mindestens 83% Identität zu den Aminosäuren 1 bis 299 von SEQ ID NO: 2 besitzt. Bevorzugt beträgt der Identitätsgrad zu den Aminosäuren 1 bis 299 von SEQ ID NO: 2 mindestens 90%, bevorzugter mindestens 95%, noch bevorzugter mindestens 97% und besonders bevorzugt mindestens 98%, unter der Bedingung, dass die jeweiligen Sequenzen Phospholipaseaktivität im Wesentlichen ohne Lipaseaktivität aufweisen.

Die erfindungsgemäßen Polypeptide mit Phospholipaseaktivität besitzen keine nennenswerte Lipaseaktivität bzw. sind im Wesentlichen ohne Lipaseaktivität. Die erfindungsgemäßen Polypeptide besitzen dabei im Wesentlichen keine für industrielle Prozesse der Ölentschleimung nachteilige Lipaseaktivität, d.h., die erfindungsgemäßen Polypeptide zeigen im Wesentlichen keine Aktivität gegen lipolytisch spaltbare Verbindungen im zu entschleimenden Öl. Dies bedeutet, dass die erfindungsgemäßen Phospholipasen unter den Bedingungen der enzymatischen Entschleimung von Speiseöl keine für diesen Prozess relevante Lipaseaktivität besitzen. Technologisch bedeutet dies, dass die erfindungsgemäßen Polypeptide mit Phospholipaseaktivität p-Nitrophenylpalmitat als Lipase-Substrat nur in einem unbedeutenden und/oder nicht nachweisbaren Ausmaß hydrolysieren. Bei den erfindungsgemäßen Polypeptiden mit Phospholipaseaktivität beträgt bevorzugt das Verhältnis Phospholipaseaktivität zu Lipaseaktivität >1.000:1, bevorzugter 5.000:1 bis 10.000:1, noch bevorzugter 7.000:1, am bevorzugtesten 7.500:1.

Der Grad der Sequenzidentität wird bevorzugt so bestimmt, dass man die Anzahl der Reste der kürzeren Sequenz, die an dem Vergleich beteiligt ist und die ein "entsprechendes" Gegenstück in der anderen Sequenz besitzt, ermittelt. Für die Zwecke der vorliegenden Erfindung wird die Identität dabei bevorzugt in üblicher Weise unter Verwendung der üblichen Algorithmen bestimmt. Erfindungsgemäß werden zum Vergleich nur die cDNAs bzw. Aminosäuren der jeweiligen reifen Proteine herangezogen. Als homologe Sequenzen wurden erfindungsgemäß ähnliche, bevorzugt identische, Sequenzgegenstücke mit Hilfe von bekannten Computerprogrammen ermittelt. Ein Beispiel für ein derartiges Programm ist das Programm Clone Manager Suite, das den Programmteil Align Plus enthält und von Scientific & Educational Software, Durham, NC, USA vertrieben wird. Dabei wird ein Vergleich zweier wie vorstehend definierter DNA- bzw. Aminosäuresequenzen durchgeführt, unter der Option local alignment entweder nach der Methode FastScan - MaxScore oder nach der Methode Needleman-Wunsch unter Beibehaltung der Defaultwerte. Speziell wurde erfindungsgemäß zur Berechnung der Identität die Programmversion "Clone Manager 7 Align Plus 5" mit den Funktionen "Compare Two Sequences/Local Fast Scan-Max Score/Compare DNA sequences", bzw. für Aminosäuren "Compare Two Sequences/Global/Compare sequences as Amino Acids" angewendet. Dabei wurden die aus den folgenden Quellen zugänglichen Algorithmen verwendet: Hirschberg, D.S. 1975. A linear space algorithm for computing maximal common subsequences. Commun Assoc Comput Mach 18:341-343; Myers, E.W. and W. Miller. 1988. Optimal alignments in linear space. CABIOS 4:1, 11-17; Chao, K-M, W.R. Pearson and W. Miller. 1992. Aligning two sequences within a specified diagonal band. CABIOS 8:5, 481-487.

Die Erfindung betrifft ferner Additions- und/oder Deletionsmoleküle der vorstehenden Polypeptide mit Phospholipaseaktivität. So kann ein erfindungsgemäß modifiziertes Polypeptid mit Phospholipaseaktivität durch Anfügen weiterer Sequenzen am N-terminalen und/oder C-terminalen Ende verlängert werden, wobei die so erhaltenen Aminosäuresequenzen noch Phospholipaseaktivität im Wesentlichen ohne Lipaseaktivität aufweisen müssen. Dadurch können Hybridmoleküle hergestellt werden, die weitere vorteilhafte Eigenschaften besitzen. Beispielsweise können Suspensionsproteine bzw. deren native Vorläuferformen in hohem Maße sekretierte Proteine angefügt werden, wodurch die Sekretionseffizienz weiter verbessert wird. Ferner können aktive Sequenzabschnitte anderer Enzyme hinzugefügt werden, um Enzyme mit mehrfacher Spezifität herzustellen. Ferner können polare oder unpolare Sequenzen angefügt werden, um die Löslichkeitseigenschaften bzw. die Membrangängigkeit des so erhaltenen Enzyms in gezielter Weise zu beeinflussen.

Erfindungsgemäß können auch Sequenzabschnitte des Polypeptids mit Phospholipaseaktivität deletiert werden unter Beibehaltung der Phospholipaseaktivität im Wesentlichen ohne Lipaseaktivität. Die Mutationen, Elongationen und Verkürzungen können in an sich bekannter Weise nach auf dem Fachgebiet gut bekannten Verfahren durchgeführt werden. Verkürzte Polypeptide zeichnen sich häufig durch eine verbesserte Sekretionshöhe im Vergleich zu den Volllängenpolypeptiden aus. Sie können auch höhere Thermostabilitäten im Vergleich zum Volllängenpolypeptid aufweisen, da sie nur den "komprimierten Kern" enthalten.

Die Erzeugung solcher Varianten ist allgemein auf dem Fachgebiet bekannt. Beispielsweise können Aminosäuresequenz-Varianten der Polypeptide durch Mutation in der DNA hergestellt werden. Verfahren zur Mutagenese und Nukleotidsequenz-Veränderung sind auf dem Fachgebiet gut bekannt (vgl. beispielsweise Tomic et al. NAR, 18:1656 (1990), Giebel and Sprtiz NAR, 18:4947 (1990)).

Hinweise über geeignete Aminosäuresubstitutionen, die die biologische Aktivität des Proteins von Interesse nicht beeinträchtigen, finden sich in dem Modell von Dayhoff et al., Atlas of Protein Sequence and Structure, Natl. Biomed. Res. Found., Washington, D.C. (1978). Konservative Substitutionen wie der Austausch einer Aminosäure gegen eine andere mit ähnlichen Eigenschaften sind bevorzugt. Diese Austausche lassen sich in zwei Hauptgruppen mit zusammen vier Untergruppen einteilen und ein Austausch in jeder Untergruppe wird als konservativer Austausch bezeichnet, der bevorzugt nicht die Aktivität oder die Faltung des Proteins beeinflusst.

| Aliphatisch | Nicht-Polar | G A P |
| | | I L V |
| | Polar und ungeladen | C S T M N Q |
| | Polar und geladen | D E |
| | | K R |
| Aromatisch | | H F W Y |

Die Ausdrücke "Protein", "Peptid" und "Polypeptid" werden im Wesentlichen austauschbar verwendet. Ein Polypeptid oder Enzym mit Phospholipaseaktivität oder eine Phospholipase soll ein Enzym bezeichnen, das die Freisetzung von Fettsäuren aus Phospholipiden, zum Beispiel Lecithinen, katalysiert. Die Phospholipaseaktivität kann unter Verwendung an sich bekannter, beliebiger Meßmethoden, bei denen eines dieser Substrate verwendet wird, bestimmt werden.

Im Zusammenhang mit den erfindungsgemäßen Polypeptiden soll der Ausdruck "Phospholipase" bzw. Phospholipase A sowohl Enzyme mit Phospholipase A1- als auch Phospholipase A2-Aktivität bezeichnen. Dabei sind Phospholipase A1 bzw. A2 definiert gemäß der Standardenzym-EC-Klassifikation als EC 3.1.1.32 bzw. 3.1.1.4.

Phospholipase B oder Lysophospholipase sind Polypeptide gemäß der Standardenzym-EC-Klassifikation EC 3.1.1.5.

Die Erfindung betrifft ferner DNA-Sequenzen, die ein Polypeptid mit Phospholipaseaktivität kodieren, die Mutationen, Modifikationen bzw. Variationen der Sequenz gemäß SEQ ID NO: 1 wie vorstehend definiert umfassen. Ferner betrifft die Erfindung auch Sequenzen, die unter stringenten Bedingungen mit den vorstehenden Sequenzen hybridisieren. Als stringente Bedingungen gelten: Hybridisierung bei 65°C, 18 h in Dextransulfat-Lösung (GenescreenPlus, DuPont), danach waschen der Filter jeweils 30 min zuerst mit 6 x SSC, zweimal 2 x SSC, zweimal 2 x SSC, 0,1% SDS und anschließend mit 0, 2 x SSC bei 65°C (Membrane transfer and detection methods, Amersham).

Ferner betrifft die Erfindung auch DNA-Sequenzen, die aufgrund der Degeneriertheit des genetischen Codes mit den vorstehenden erfindungsgemäßen Sequenzen verwandt sind. Die Degeneriertheit des genetischen Codes kann sich dabei aufgrund natürlicher Degeneriertheit oder aufgrund eines speziell gewählten Kodongebrauchs ergeben. Natürlich vorkommende allelische Varianten können unter Verwendung gut bekannter Techniken der Molekularbiologie, wie zum Beispiel der Polymerasekettenreaktion (PCR) und Hybridisierungstechniken, identifiziert werden.

Die Erfindung betrifft ferner ein Verfahren zur Herstellung eines Polypeptids mit Phospholipaseaktivität nach rekombinanten Techniken umfassend die Züchtung rekombinanter prokaryotischer und/oder eukaryotischer Wirtszellen, die eine erfindungsgemäße DNA-Sequenz enthalten unter Bedingungen, die die Expression des Enzyms fördern, sowie die anschließende Gewinnung des Enzyms. Die Erfindung betrifft ferner die Verwendung der erfindungsgemäßen Polynukleotidsequenzen zur Herstellung von Sonden zum Auffinden ähnlicher Sequenzen, die entsprechende Enzyme kodieren, in anderen Organismen sowie zur Transformation von Wirtszellen.

Eine DNA-Sequenz, die ein erfindungsgemäßes Polypeptid kodiert, kann verwendet werden, um beliebige Wirtszellen, wie beispielsweise Zellen von Pilzen, Hefen, Bakterien, Pflanzen oder Säugern zu transformieren. Derart transformierte Zellen zeichnen sich durch eine Sekretion der erfindungsgemäßen Phospholipase aus. Das so produzierte Phospholipaseenzym bewirkt eine effiziente Hydrolyse der Fettsäuren aus Phospholipiden.

Die Erfindung betrifft auch Expressionskassetten, die zur Einschleusung der eine erfindungsgemäße Phospholipase kodierenden DNA-Sequenz bzw. eines offenen Leserasters in eine Wirtszelle verwendet werden können. Sie umfassen bevorzugt eine Transkriptionsstartregion, die mit dem offenen Leseraster verknüpft ist. Eine derartige Expressionskassette kann eine Vielzahl von Restriktionsspaltstellen zur Insertion des offenen Leserasters und/oder anderer DNAs, z.B. einer Transkriptions-Regulator-Region und/oder selektierbare Markergene enthalten. Die Transkriptionskassette umfasst in der 5'→3'-Richtung der Transkription eine Transkriptions- und Translationsstartregion, die DNA-Sequenz von Interesse und eine Transkriptions- und Translationsstopregion, die in einer mikrobiellen Zelle funktionell ist. Die Terminationsregion kann bezüglich der Transkriptioninitiationsregion nativ sein, kann bezüglich der DNA-Sequenz von Interesse nativ sein oder kann von einer beliebigen anderen Quelle abgeleitet sein.

Der Ausdruck "offenes Leseraster" (ORF) bezeichnet die Aminosäure-Sequenz, die zwischen den Translationsstart- und -Stop-Kodons einer kodierenden Sequenz kodiert wird. Die Ausdrücke "Start-Kodon" und "Stop-Kodon" bezeichnen eine Einheit aus drei benachbarten Nukleotiden (Kodons) in einer kodierenden Sequenz, die den Kettenstart und -stop der Proteinsynthese (mRNA-Translation) spezifizieren.

"Funktionelle Verknüpfung" bezeichnet im Zusammenhang mit einer Nukleinsäure eine Verbindung als Teil des gleichen Nukleinsäuremoleküls in geeigneter Positionierung und Orientierung zum Transkriptionsstart des Promotors. DNA in funktioneller Verknüpfung an einen Promotor befindet sich unter der Transkriptions-Initiations-Regulation des Promotors. Kodierende Sequenzen können funktionell mit der Regulatorsequenz in Sense- oder Antisense-Orientierung verknüpft sein. Bei Bezugnahme auf Polypeptide bedeutet funktionelle Verknüpfung die Verbindung als Teil desselben Polypeptids, d.h. über Peptidylbindungen.

Erfindungsgemäß können beliebige Promotoren verwendet werden. Promotor bezeichnet die Nukleotidsequenz üblicherweise stromaufwärts (5') bezüglich der kodierenden Sequenz und kontrolliert die Expression der kodierenden Sequenz, indem die Erkennung für die RNA-Polymerase und anderer Faktoren, die für die richtige Transkription erforderlich ist, bereitgestellt wird. Der erfindungsgemäß verwendete Promotor kann einen Minimalpromotor umfassen, d.h. eine kurze DNA-Sequenz aus einer TATA-Box und anderen Sequenzen, die die Transkriptionsstartstelle spezifizieren, an die Regulatorelemente zur Kontrolle der Expression angefügt sind.

Der erfindungsgemäß verwendete Promotor kann auch eine Nukleotidsequenz umfassen, die einen Minimalpromotor und Regulatorelemente umfasst, der die Expression einer kodierenden Sequenz oder funktionellen RNA kontrollieren kann. Dieser Typ von Promotorsequenz besteht aus proximalen und distalen stromaufwärts gelegenen Elementen, wobei die zuletzt genannten Elemente oft als Enhancer bezeichnet werden. Folglich ist ein Enhancer eine DNA-Sequenz, die die Promotor-Aktivität stimulieren kann und kann ein dem Promotor innewohnendes Element oder ein insertiertes heterologes Element sein, um die Expressionshöhe oder Gewebsspezifität eines Promotors zu verstärken. Er kann in beiden Orientierungen funktionieren und kann selbst bei stromaufwärtiger oder stromabwärtiger Platzierung von dem Promotor funktionieren. Sowohl Enhancer als auch andere stromaufwärts gelegene Promotor-Elemente binden sequenzspezifisch DNA-bindende Proteine, die ihre Wirkungen vermitteln. Promotoren können in ihrer Gesamtheit von einem nativen Gen abgeleitet sein oder können aus verschiedenen Elementen zusammengesetzt sein, die von verschiedenen natürlich vorkommenden Promotoren abgeleitet sind oder können sogar aus synthetischen DNA-Segmenten zusammengesetzt sein. Ein Promotor kann auch DNA-Sequenzen enthalten, die an der Bindung von Proteinfaktoren beteiligt sind, die die Effizienz der Transkriptionsinitiation als Antwort auf physiologische oder entwicklungsbedingte Bedingungen kontrollieren.

Promotorelemente, insbesondere TATA-Elemente, die inaktiv sind oder eine stark verminderte Promotor-Aktivität in Abwesenheit einer stromaufwärtigen Aktivierung besitzen, werden als Minimalpromotoren oder Kernpromotoren bezeichnet. In Gegenwart eines geeigneten Transkriptionsfaktors bzw. geeigneter Transkriptionsfaktoren liegt die Funktion des Minimalpromotors in der Ermöglichung der Transkription. Ein Minimal- oder Kernpromotor besteht somit nur aus allen Grundelementen, die für die Transkriptionsinitiation notwendig sind, z. B. einer TATA-Box und/oder einem Initiator.

Die Erfindung betrifft auch erfindungsgemäße DNA-Sequenzen enthaltende Vektorkonstrukte. Diese Vektorkonstrukte umfassen beliebige Plasmide, Cosmide, Phagen und andere Vektoren in doppelsträngiger oder einzelsträngiger, linearer oder zirkulärer Form, die gegebenenfalls selbst transmittierbar oder mobilisierbar sein können und die einen prokaryotischen oder eukaryotischen Wirt entweder durch Integration in das zelluläre Genom transformieren können oder die extrachromosomal vorliegen (z.B. autonom replizierende Plasmide mit einem Replikationsorigin).

Vektoren, Plasmide, Cosmide, künstliche Hefechromosomen (YACs), künstliche Bakterienchromosomen (BACs) und DNA-Segmente zur Verwendung zur Transformation von Zellen umfassen allgemein die die erfindungsgemäße Phospholipase kodierende DNA sowie eine andere DNA, wie cDNA, ein Gen oder Gene, die in die Zellen eingeschleust werden sollen. Diese DNA-Konstrukte können weitere Strukturen wie Promotoren, Enhancer, Polylinker oder auch Regulatorgene, so weit erforderlich, umfassen. Eines der DNA-Segmente oder Gene, das bzw. die für die zelluläre Einschleusung ausgewählt wurde bzw. wurden, kodiert/kodieren zweckdienlicherweise ein Protein, das in den so erhaltenen transformierten (rekombinanten Zellen) exprimiert wird, was zu einem screenbaren oder selektierbaren Merkmal führt und/oder der transformierten Zelle einen verbesserten Phänotyp verleiht.

Die Konstruktion von Vektoren, die erfindungsgemäß verwendet werden können, ist einem Fachmann auf dem Gebiet angesichts der vorstehenden Offenbarung und des allgemeinen Fachwissens bekannt (vgl. z. B. Sambrook et al., Molecular Cloning: A Laboratory Manual (2. Aufl., Cold Spring Harbor Laboratory Press, Plainview, N.Y. (1989)).

Eine erfindungsgemäße Expressionskassette kann eine oder mehrere Restriktionsschnittstelle(n) enthalten, um das die Phospholipase kodierende Polynukleotid unter die Regulation einer Regulatorsequenz zu stellen. Die Expressionskassette kann auch ein Terminationssignal in funktioneller Verknüpfung mit dem Polynukleotid sowie Regulatorsequenzen, die für die ordnungsgemäße Translation des Polynukleotids benötigt werden, enthalten. Die Expressionskassette, die das erfindungsgemäße Polynukleotid enthält, kann chimär sein, d.h. mindestens eine ihrer Komponenten ist bezogen auf mindestens eine der anderen Komponenten heterolog. Die Expression des Polynukleotids in der Expressionskassette kann unter der Kontrolle eines konstitutiven Promotors, eines induzierbaren Promotors, eines regulierten Promotors, eines viralen Promotors oder eines synthetischen Promotors stehen.

Die Vektoren können bereits Regulatorelemente enthalten, z.B. Promotoren, oder die erfindungsgemäßen DNA-Sequenzen können so manipuliert werden, dass sie solche Elemente enthalten. Geeignete Promotorelemente, die verwendet werden können, sind auf dem Fachgebiet bekannt und sind beispielsweise für Trichoderma reesei der cbh1- oder der cbh2-Promotor, für Aspergillus oryzae der amy-Promotor, für *Aspergillus* niger der xyl-, glaA-, alcA-, aphA-, tpiA-, gpdA-, sucl- und der pkiA-Promotor. Geeignete Promotorelemente, die zur Expression in Hefe verwendet werden können, sind auf dem Fachgebiet bekannt und sind beispielsweise der pho5-Promotor oder der gap-Promotor zur Expression in Saccharomyces cerevisiae und für Pichia pastoris, z.B. der aoxl-Promotor oder der fmd-Promotor oder der mox-Promotor für H. polymorpha.

DNA, die zur Einschleusung in Zellen geeignet ist, kann neben der erfindungsgemäßen DNA auch DNA umfassen, die aus einer beliebigen Quelle abgeleitet wurde oder daraus isoliert ist. Ein Beispiel für eine abgeleitete DNA ist eine DNA-Sequenz, die als nützliches Fragment in einem gegebenen Organismus identifiziert wurde und die dann in im Wesentlichen reiner Form chemisch synthetisiert wurde. Ein Beispiel für eine solche DNA ist eine geeignete DNA-Sequenz, die beispielsweise unter Verwendung von Restriktionsendonukleasen erhalten wurde, so dass sie weiter erfindungsgemäß manipuliert, beispielsweise amplifiziert werden kann. Zu diesen zählt unter anderem das als Markergen verwendbare amdS-Gen aus *Aspergillus nidulans* und seine regulatorischen Sequenzen, sowie Polylinker.

Eine derartige DNA wird üblicherweise als rekombinante DNA bezeichnet. Daher umfasst eine geeignete DNA vollständig synthetische DNA, semisynthetische DNA, aus biologischen Quellen isolierte DNA und von eingeschleuster RNA abgeleitete DNA. Im Allgemeinen ist die eingeschleuste DNA kein ursprünglicher Bestandteil des Genotyps der Empfänger-DNA, aber erfindungsgemäß kann auch ein Gen aus einem gegebenen Genotyp isoliert und eventuell verändert werden und anschließend können Mehrfachkopien des Gens in den gleichen Genotyp eingeschleust werden, z. B. um die Produktion eines gegebenen Genprodukts zu verstärken.

Die eingeschleuste DNA umfasst ohne Beschränkung DNA aus Genen, wie beispielsweise aus Bakterien, Hefen, Pilzen oder Viren. Die eingeschleuste DNA kann modifizierte oder synthetische Gene, Teile von Genen oder chimäre Gene einschließlich Gene aus dem gleichen oder einem unterschiedlichen Genotyp umfassen. Hierzu kann z. B. auch DNA der Plasmide pUC18, pUC19 gehören.

Die zur Transformation erfindungsgemäß verwendete DNA kann zirkulär oder linear, doppelsträngig oder einzelsträngig sein. Im Allgemeinen liegt die DNA in Form einer chimären DNA wie eine Plasmid-DNA vor, die auch kodierende Regionen enthält, die von Regulatorsequenzen flankiert ist und die Expression der in der transformierten Zelle vorhandenen rekombinanten DNA unterstützen. Beispielsweise kann die DNA selbst einen Promotor enthalten oder daraus bestehen, der in einer Zelle aktiv ist, der von einer Quelle, die sich von der Zelle unterscheidet, abgeleitet ist oder es kann ein Promotor verwendet werden, der bereits in der Zelle, d.h. der Transformationszielzelle, vorhanden ist.

Im Allgemeinen ist die eingeschleuste DNA relativ klein, weniger als etwa 30 kb, um die Empfindlichkeit gegenüber physikalischem, chemischem oder enzymatischem Abbau zu minimieren, der mit der Größe der DNA zunimmt.

Die Selektion eines geeigneten Expressionsvektors hängt von den Wirtszellen ab. Hefe- oder Pilzexpressionsvektoren können einen Replikationsorigin, einen geeigneten Promotor und Enhancer umfassen, und auch beliebige notwendige Ribosomenbindungsstellen, Polyadenylierungsstellen, Splice-Donor und -Akzeptor-Stellen, Transkriptionsterminationssequenzen und nicht-transkribierte 5'-flankierende Sequenzen umfassen.

Beispiele für geeignete Wirtszellen sind: Pilzzellen der Gattung *Aspergillus, Rhizopus, Trichoderma, Neurospora, Mucor, Penicillium,* etc., wie beispielsweise Hefen der Gattungen *Kluyveromyces, Saccharomyces, Schizosaccharomyces, Trichosporon, Schwanniomyces, Hansenula, Pichia* und dergleichen. Geeignete Wirtssysteme sind beispielsweise Pilze wie *Aspergilli,* z.B. *Aspergillus niger* (ATCC 9142) oder *Aspergillus ficuum* (NRLL 3135) oder *Trichoderma* (z.B. *Trichoderma reesei* QM6a) und Hefen wie *Saccharomyces,* z.B. *Saccharomyces cerevisiae* oder *Pichia,* wie z.B. *Pichia pastoris* oder *Hansenula,* z.B. *H. polymorpha* (DSMZ 70277). Derartige Mikroorganismen können von anerkannten Hinterlegungsstellen, z.B. der American Type Culture Collection (ATCC), dem Centraalbureau voor Schimmelcultures (CBS) oder der Deutschen Sammlung für Mikroorganismen und Zellkulturen GmbH (DSMZ) oder beliebigen anderen Hinterlegungsstellen erhalten werden.

Die Expressionskassette kann in der 5'-3'-Transkriptionsrichtung eine Transkriptions-und Translationstartregion des erfindungsgemäßen Polynukleotids und eine Transkriptions- und Terminationsregion, die in vivo oder in vitro funktionell ist, enthalten. Die Terminationsregion kann bezüglich der Transkriptionsinitiationsregion nativ sein oder kann bezüglich des Polynukleotids nativ oder anderer Herkunft sein. Die Regulatorsequenzen können stromaufwärts (5' nicht-kodierende Sequenzen), innerhalb (Intron) oder stromabwärts (3' nicht-kodierende Sequenzen) einer kodierenden Sequenz lokalisiert sein und die Transkription, das RNA-Processing oder die Stabilität und/oder die Translation der assoziierten kodierenden Sequenz beeinflussen. Regulatorsequenzen können ohne Beschränkung Enhancer, Promotoren, Repressorbindungsstellen, Translationsleadersequenzen, Introns oder Polyadenylierungsignalsequenzen umfassen. Sie können natürliche und synthetische Sequenzen sowie Sequenzen umfassen, die eine Kombination aus synthetischen und natürlichen Sequenzen sind.

Der erfindungsgemäß verwendete Vektor kann auch geeignete Sequenzen zur Amplifikation der Expression umfassen.

Beispiele für Promotoren, die erfindungsgemäß verwendet werden können, sind Promotoren, von denen bekannt ist, dass sie die Expression in den eukaryotischen Zellen kontrollieren. Beliebige Promotoren mit der Fähigkeit zur Expression in Fadenpilzen können verwendet werden. Beispiele sind ein Promotor, der stark durch Stärke oder Zellulose induziert wird, z. B. ein Promotor für Glucoamylase oder α-Amylase aus der Gattung *Aspergillus* oder für Cellulase (Cellobiohydrolase) aus der Gattung *Trichoderma,* ein Promotor für Enzyme im glykolytischen Stoffwechselweg, wie beispielsweise Phosphoglyceratkinase (PGK) und Glycerinaldehyd-3-phosphat-dehydrogenase (GPD), etc.. Bevorzugt ist der Cellobiohydrolase-I-, der Cellobiohydrolase-II-, der Amylase-, der Glucoamylase-, der Xylanase- oder der Enolase-Promotor.

Zusätzlich zur Verwendung eines speziellen Promotors können andere Typen von Elementen die Expression von Transgenen beeinflussen. Insbesondere wurde gezeigt, dass Introns das Potenzial zur Verstärkung der Transgenexpression besitzen.

Die Expressionskassette kann noch weitere Elemente umfassen, beispielsweise solche, die durch endogene oder exogene Elemente wie Zinkfinger-Proteine, einschließlich natürlich vorkommender Zinkfinger-Proteine oder chimärer Zinkfinger-Proteine, reguliert werden können.

Die erfindungsgemäß verwendete Expressionskassette kann ferner Enhancer-Elemente oder stromaufwärtige Promotor-Elemente enthalten.

Vektoren zur erfindungsgemäßen Verwendung können so konstruiert werden, dass sie ein Enhancer-Element enthalten. Die erfindungsgemäßen Konstrukte umfassen somit das Gen von Interesse zusammen mit einer 3'-DNA-Sequenz, die als Signal wirkt, um die Transkription zu terminieren und die Polyadenylierung der so erhaltenen mRNA zu erlauben. Es können beliebige Signalsequenzen verwendet werden, die die Sekretion aus dem gewählten Wirtsorganismus ermöglichen. Eine bevorzugte Signalsequenz ist die Phospholipase-Signalsequenz aus *Aspergillus fumigatus* oder daraus abgeleitete Signalsequenzen für die Sekretion aus filamentösen Pilzen.

Es kann auch eine spezielle Leadersequenz verwendet werden, da die DNA-Sequenz zwischen der Transkriptionsstartstelle und dem Start der kodierenden Sequenz, d.h. der nicht-translatierten Leadersequenz die Genexpression beeinflussen kann. Bevorzugte Leadersequenzen umfassen Sequenzen, die die optimale Expression des angehefteten Gens steuern, d.h. sie umfassen eine bevorzugte Consensus-Leader-Sequenz, die die mRNA-Stabilität erhöht oder erhält und eine ungeeignete Translationsinitiation verhindert. Die Wahl solcher Sequenzen ist einem Fachmann auf dem Gebiet gut bekannt.

Um die Möglichkeit, der Identifikation der Transformanten zu verbessern, kann ein selektierbares oder screenbares Markergen in die Expressionskassette aufgenommen werden. Derartige Markergene sind einem Fachmann auf dem Gebiet gut bekannt.

Die Expressionskassette oder ein Vektorkonstrukt, das die Expressionskassette enthält, wird in eine Wirtszelle eingeführt. Eine Vielzahl von Techniken ist verfügbar und einem Fachmann auf dem Gebiet zur Einschleusung von Konstrukten in eine Wirtszelle gut bekannt. Die Transformation mikrobieller Zellen kann unter Verwendung von Polyethylenglykol, Calciumchlorid, viraler Infektion, DEAE-Dextran, Phageninfektionen, Elektroporation und anderen auf dem Fachgebiet bekannten Methoden durchgeführt werden. Die Transformation von Pilzen kann nach Penttilä et al., Gene 61:155-164, 1987 durchgeführt werden. Die Einschleusung eines rekombinanten Vektors in Hefen kann nach an sich bekannten Verfahren einschließlich Elektroporation, Verwendung von Sphäroplasten, Lithiumacetat und dergleichen durchgeführt werden.

Sobald die erfindungsgemäße Expressionskassette bzw. DNA-Sequenz erhalten ist, kann sie in Vektoren nach an sich bekannten Verfahren eingefügt werden, um das kodierte Polypeptid in geeigneten Wirtssystemen zu überexprimieren. Jedoch können auch DNA-Sequenzen als solche verwendet werden, um geeignete Wirtssysteme der Erfindung zu transformieren, um eine Überexpression des codierten Polypeptids zu erzielen.

Sobald eine erfindungsgemäße DNA-Sequenz in einer geeigneten Wirtszelle in einem geeigneten Medium exprimiert wurde, kann die kodierte Phospholipase entweder aus dem Medium, falls die Phospholipase in das Medium sezerniert wird oder aus dem Wirtsorganismus, falls die Phospholipase intrazellulär z. B. im periplasmatischen Raum vorhanden ist, nach an sich bekannten Verfahren aufkonzentriert und/oder isoliert werden. Bekannte Verfahren der Abtrennung der unlöslichen Bestandteile des Kulturmediums und der Biomasse gefolgt von Verfahren zur Aufkonzentrierung der Phospholipase können zur Herstellung von konzentrierten Phospholipaselösungen oder als Vorbereitung zur Trocknung der Phospholipase angewendet werden. Beispielsweise können Filtrationsverfahren oder Zentrifugationsverfahren zur Abtrennung der unlöslichen Bestandteile verwendet werden gefolgt von Ultrafiltrationsverfahren zur Aufkonzentrierung oder es werden Cross-flow-Filtrationsverfahren angewendet. Die Trocknung kann durch Gefrier- und Sprühtrocknen, Granulationsverfahren, Extrudierung oder andere Verfahren erfolgen. Bekannte Verfahren der Proteinreinigung können verwendet werden, um die erfindungsgemäßen Phospholipasen zu isolieren. Beispielsweise können verschiedene chromatographische oder gelchromatographische Verfahren einzeln oder in Kombination verwendet werden. In Abhängigkeit von der verwendeten Wirtszelle bei einem rekombinanten Produktionsverfahren kann das erfindungsgemäße Enzym kovalent durch Glykosilierung modifiziert sein oder nicht. In eukaryotischen Zellen dient die Glykosilierung der sezernierten Proteine dazu, die Proteinfaltung, die Konformationsstabilität, die thermische Stabilität und die Resistenz gegenüber Proteolyse zu modulieren. Im Hinblick auf eine spezifische Anwendung der Phospholipase kann eine glykosilierte Variante des Enzyms gegenüber einer nicht-glykosilierten Variante bevorzugt sein.

Die Erfindung betrifft auch isolierte oder im Wesentlichen gereinigte Nukleinsäure-oder Proteinzusammensetzungen. Dabei bezeichnet ein isoliertes und gereinigtes Polynukleotid/Polypeptid bzw. Segment davon ein Polynukleotid bzw. Polypeptid bzw. Segment davon, das isoliert aus seiner nativen Umgebung und in gereinigter Form zur weiteren Verwendung vorliegt. Ein isoliertes Polynukleinsäuresegment oder Polypeptid kann in gereinigter Form vorliegen oder kann in einer nicht nativen Umgebung vorliegen, wie beispielsweise in einer transgenen Wirtszelle. Beispielsweise ist ein isoliertes oder gereinigtes Polynukleotidsegment oder Protein oder ein biologisch aktiver Teil davon im Wesentlichen frei von weiterem zellulärem Material oder Kulturmedium bei Produktion nach rekombinanten Techniken oder im Wesentlichen frei von chemischen Vorläufern oder anderen chemischen Verbindungen. Bevorzugt ist ein isoliertes Polynukleotid frei von Sequenzen (bevorzugt Protein-kodierenden Sequenzen) die natürlicherweise die Nukleinsäure (d.h. Sequenzen, die an den 5'- und 3'-Enden der Nukleinsäure lokalisiert sind) in der genomischen DNA des Organismus aus dem die Nukleinsäure stammt, flankieren. Beispielsweise kann gemäß verschiedenen Ausführungsformen das isolierte Nukleinsäuremolekül weniger als etwa 5 kb, 4 kb, 3 kb, 2 kb, 1 kb, 0,5 kb oder 0,1 kb Nukleotidsequenzen enthalten, die natürlicherweise das Nukleinsäuremolekül in der genomischen DNA der Zelle, aus der die Nukleinsäure abgeleitet ist, flankieren. Ein Protein, das im Wesentlichen frei von zellulärem Material ist, umfasst Zusammensetzungen von Protein oder Polypeptid mit weniger als etwa 70%, 50%, 30%, 20%, 10%, 5% (bezogen auf das Trockengewicht) von verunreinigendem Protein. Wenn das erfindungsgemäße Protein oder ein biologisch aktives Fragment davon rekombinant produziert wird, umfasst bevorzugt das Kulturmedium weniger als etwa 70%, 50%, 30%, 20%, 10% oder 5% (bezogen auf das Trockengewicht) der chemischen Vorläufer oder nicht-proteinartigen chemischen Substanzen.

Die Erfindung betrifft auch Phospholipasezusammensetzungen, die das erfindungsgemäße Polypeptid enthalten. Im Allgemeinen sind Phospholipasezusammensetzungen flüssig oder trocken. Flüssige Zusammensetzungen enthalten das Phospholipaseenzym bevorzugt in einer gereinigten oder angereicherten Form. Jedoch können auch Hilfsstoffe wie beispielsweise ein Stabilisator und/oder Glycerin, Sorbit oder Monopropylenglykol, Additive wie Salze, Zucker, Konservierungsstoffe, Mittel zur Einstellung des pH-Werts und Proteine zugesetzt werden. Typische Flüssigzusammensetzungen sind wässrige oder ölige Aufschlämmungen.

Trockenzusammensetzungen können gefriergetrocknete, sprühgetrocknete, granulierte oder extrudierte Zusammensetzungen sein, die ausschließlich das Enzym enthalten können. Trockenzusammensetzungen können Granulate sein, die leicht beispielsweise mit Nahrungsmitteln oder Futterkomponenten gemischt werden können oder bevorzugter eine Komponente eines Prämix bilden. Die Teilchengröße des Enzymgranulats ist bevorzugt mit der der anderen Komponenten des Gemisches kompatibel. Dies ermöglicht sichere und zweckdienliche Mittel, um Enzyme beispielsweise in prozessierte Nahrungsmittel, Prämixe oder Tierfutter einzuarbeiten.

Trockenzusammensetzungen können auch andere Additive wie z.B. Salze, insbesondere Phosphatsalze und deren Anhydroformen und Stabilisierungsmittel wie Polyvinylpyrrolidon etc. enthalten um bestimmte Bedingungen, wie z.B. den pH-Wert in der Anwendung zu regulieren.

Auf ähnliche Weise kann ein Nahrungsmitteladditiv gemäß dieser Ausführungsform der vorliegenden Erfindung mit anderen Nahrungsmittelkomponenten vereinigt werden, wodurch verarbeitete Nahrungsmittelprodukte erzeugt werden. Solche anderen Nahrungsmittelkomponenten umfassen ein oder mehrere Enzymsupplemente, Vitamine, Mineralien und Spurenelemente. Das so erhaltene kombinierte Nahrungsergänzungsmittel kann dann in einer geeigneten Menge mit anderen Nahrungsmittel-Komponenten wie Getreide und Pflanzenproteinen vermischt werden, um ein verarbeitetes Nahrungsmittel zu ergeben. Die Verarbeitung dieser Komponenten zu einem verarbeiteten Nahrungsmittel kann unter Verwendung an sich bekannter Verarbeitungsvorrichtungen durchgeführt werden.

In einer bevorzugten Ausführungsform umfassen die erfindungsgemäßen Phospholipasezusammensetzungen zusätzlich eine wirksame Menge eines oder mehrerer Enzyme für Nahrungs- oder Futtermittel oder für die Anwendung in Vorstufen zur Herstellung von Nahrungs- oder Futtermitteln, oder bei der Anwendung in der Textilindustrie, bevorzugt ausgewählt aus alpha-Galactosidasen, beta-Galactosidasen, Laccasen, anderen Phospholipasen, Phosphatasen, Endoglucanasen, insbesondere endo-beta-1,4-Glucanasen, endo-beta-1,3(4)-Glucanasen, endo-1,2-beta-Glucanasen und endo-1,3-alpha-Glucanasen, Cellulasen, Xylosidasen, Galactanasen, insbesondere Arabinogalactan-endo-1,4-beta-Galactosidasen und Arabinogalactan-endo-1,3-beta-Galactosidasen, Pectinabbauenden Enzymen, insbesondere Pectinasen, Pectinesterasen, Pectinlyasen, Polygalacturonasen, Arabananasen, Rhamnogalacturonasen, Rhamnogalacturonanacetylesterasen, Rhamnogalacturonan-alpha-Rhamnosidasen, Pectatlyasen und alpha-Galacturonidasen, Mannanasen, beta-Mannosidasen, Mannanacetylesterasen, Xylanacetylesterasen, Proteasen, Xylanasen, Arabinoxylanasen, lipolytischen Enzymen wie Lipasen, Digalactosid-Diglycerid Esterasen und Cutinasen, und andere Enzyme wie Laccasen und Transglutaminasen.

Die erfindungsgemäßen Phospholipasen können für eine Vielzahl von Anwendungen verwendet werden. Beispiele hierfür sind Anwendungen beim Backen und in der Tierernährung sowie bei der Herstellung von Kraftstoffen aus erneuerbaren Energiequellen, zum Beispiel Rapssamen, bzw. in der Verarbeitung von Textilrohstoffen.

Eine bevorzugte Anwendung ist die Verwendung der erfindungsgemäßen Polypeptide mit Phospholipaseaktivität in Verfahren zur Entschleimung von pflanzlichem Öl. Dabei wird zum Beispiel das zu entschleimende Speiseöl mit einem erfindungsgemäßen Polypeptid behandelt, wodurch der Hauptteil der Phospholipide hydrolysiert wird, und anschließend wird die die hydrolysierten Phospholipide enthaltende wässrige Phase von dem Öl abgetrennt. Ein derartiges Verfahren eignet sich besonders zur Reinigung von Speiseölen, die Phospholipide enthalten, zum Beispiel von Pflanzenölen wie Sojabohnenöl, Rapssamenöl und Sonnenblumenöl.

Vor der Phospholipasebehandlung wird das Öl bevorzugt vorbehandelt, um Schleimstoffe zu entfernen, beispielsweise durch Feuchtraffinieren. Typischerweise enthält das Öl 50 bis 850 ppm Phosphor als Phospholipid zu Beginn der Behandlung mit der erfindungsgemäßen Phospholipase. Nach der Behandlung beträgt der Phosphorwert typischerweise zwischen 2 und 10 ppm.

Die Phospholipasebehandlung wird üblicherweise so durchgeführt, dass die Phospholipase in einer wässrigen Lösung dispergiert wird, bevorzugt als Tröpfchen mit einem durchschnittlichen Durchmesser von <10 $\mu$m. Die Menge an Wasser beträgt bevorzugt 0,5 bis 5 Gew.-% (w/w) bezogen auf das Öl. Gegebenenfalls kann ein Emulgator zugesetzt werden. Es kann mechanisch gerührt werden, um eine Emulsion aufrechtzuerhalten. Die Behandlung mit Phospholipase kann bei einem pH-Wert im Bereich von etwa 3,5 bis etwa 5,0 durchgeführt werden. Der Verfahrens-pH-Wert kann im Bereich von etwa 3,5 bis etwa 5, bevorzugt 3,8 bis 4,5 und am bevorzugtesten 4,0 bis 4,2 liegen, um die Leistungsfähigkeit des Enzyms zu maximieren. Der pH-Wert kann beispielsweise durch Zugabe von Zitronensäure, einem Citratpuffer, Phosphorsäure oder Salzsäure eingestellt werden. Eine geeignete Temperatur beträgt allgemein 30°-70°C, bevorzugt 45°-65°C und am bevorzugtesten 55°-62°C. Die Reaktionszeit beträgt typischerweise 1 bis 12 Stunden, bevorzugt 2 bis 6 Stunden. Eine geeignete Enzymdosierung beträgt üblicherweise 120 bis 3000 Einheiten pro kg Öl, bevorzugt 250 bis 2000 und am bevorzugtesten 750 bis 1500 Einheiten pro kg Öl.

Die Phospholipasebehandlung kann chargenweise, zum Beispiel in einem Tank unter Rühren, durchgeführt werden oder kann kontinuierlich sein, beispielsweise in einer Reihe von Tankreaktoren unter Rühren.

An die Phospholipasebehandlung schließt sich die Abtrennung einer wässrigen Phase und einer Ölphase an. Die Abtrennung kann durch herkömmliche Mittel, zum Beispiel Zentrifugation, durchgeführt werden. Die wässrige Phase enthält Phospholipasen und das Enzym kann zur Verbesserung der Ökonomie des Verfahrens erneut verwendet werden.

Die Behandlung kann unter Verwendung an sich bekannter Verfahren durchgeführt werden.

Die erfindungsgemäße Phospholipase kann auch vorteilhafterweise zur Herstellung von Teig und Backwaren verwendet werden, wobei in den Teig eine wirksame Menge eines erfindungsgemäßen Polypeptids eingearbeitet wird. Durch den Zusatz eines erfindungsgemäßen Polypeptids mit Phospholipaseaktivität können eine oder mehrere Eigenschaften des Teigs oder des aus dem Teig erhaltenen Backprodukts, verglichen mit einem Teig oder Backprodukt ohne Zusatz eines erfindungsgemäßen Polypeptids mit Phospholipaseaktivität, verbessert werden.

Bei der Teigbereitung unter Verwendung der erfindungsgemäßen Phospholipase kann die Phospholipase dem Teig selbst, jedem beliebigen Inhaltsstoff, aus dem der Teig hergestellt wird, und/oder einem Gemisch aus Teiginhaltsstoffen, aus denen der Teig hergestellt wird, zugesetzt werden. Ein erfindungsgemäßes Polypeptid mit Phospholipaseaktivität kann somit in jeder Stufe der Teigherstellung als solcher zugesetzt werden oder kann in ein, zwei oder mehreren Stufen zugesetzt werden. Eine wirksame Menge soll hier eine Menge an Phospholipase bezeichnen, die ausreicht einen messbaren Effekt auf mindestens eine Eigenschaft von Interesse des Teigs und/oder des Backprodukts hervorzurufen.

Der Ausdruck "verbesserte Eigenschaft" ist hier als jede Eigenschaft des Teigs und/oder eines Produkts, das aus dem Teig erhalten wird, insbesondere einer Backware, definiert, die durch die Wirkung der Phospholipase bezogen auf den Teig oder das Produkt, dem die erfindungsgemäße Phospholipase nicht zugesetzt wurde, verbessert wurde. Die verbesserte Eigenschaft kann beispielsweise umfassen: erhöhte Teigstärke, erhöhte Elastizität des Teigs, verbesserte Stabilität des Teigs, verringerte Klebrigkeit des Teigs, verbesserte Extensibilität des Teigs, verbesserte Maschinengängigkeit des Teigs, erhöhtes Volumen des Backprodukts, verbesserte Krumenstruktur des Backprodukts, verbesserte Weichheit des Backprodukts, verbessertes Aroma des Backprodukts und/oder verzögertes Altbackenwerden des Backprodukts. Verfahren zur Bestimmung dieser Eigenschaften sind im Stand der Technik gut bekannt.

Ein Teig wird hier definiert als Gemisch aus Mehl und anderen Inhaltsstoffen, das fest genug ist um geknetet oder gewalzt zu werden. Der Teig kann frisch gefroren, vorgegart oder vorgebacken sein.

Der Ausdruck "Backprodukt" bezeichnet hier jedes Produkt, das aus einem Teig hergestellt wird und entweder einen weichen oder krossen Charakter besitzt. Beispiele für Backprodukte, die unter Verwendung einer erfindungsgemäßen Phospholipase hergestellt werden können, sind beispielsweise Brot (insbesondere Weißbrot, Vollkornbrot oder Roggenbrot), typischerweise in Form von Laiben oder Stangenbrot vom Typ französisches Baguette, Pasta, Pitabrot, Tortillas, Tacos, Kuchen, Pfannkuchen, Kekse und Kleingebäck, gekochtes Brot, doppelt gebackenes Brot und dergleichen.

Bei der Herstellung dieser Backwaren kann das erfindungsgemäße Polypeptid mit Phospholipaseaktivität und/oder ein oder mehrere weitere Enzyme in beliebigen Formulierungen zugesetzt werden, die für die jeweilige Verwendung geeignet sind, zum Beispiel in trockener Form, als Flüssigkeit oder als Prämix. Ferner können ein oder mehrere weitere Enzyme dem Teig ebenfalls zugesetzt werden. Diese weiteren Enzyme können beliebigen Ursprungs sein und zum Beispiel von Säugetieren und Pflanzen abstammen. Bevorzugt sind sie mikrobiellen Ursprungs und stammen besonders bevorzugt von Bakterien oder Pilzen ab.

Gemäß einer bevorzugten Ausführungsform können die weiteren Enzyme Amylasen wie α-Amylase (geeignet zur Erzeugung von Zuckern, die von Hefe fermentierbar sind, und zur Verzögerung des Altbackenwerdens) oder β-Amylase, Cyclodextringlucanotransferase, Peptidase, insbesondere eine Exopeptidase (geeignet zur Verstärkung des Aromas), Transglutaminase, Lipase (geeignet zur Modifikation der in dem Teig oder den Teigbestandteilen vorhandenen Lipide um den Teig weicher zu machen), Phospholipase (nützlich zur Modifikation der Lipide, die in dem Teig oder in den Teigbestandteilen vorhanden sind, um den Teig weicher zu machen und die Gasretention in dem Teig zu verbessern), Cellulase, Hemicellulase, insbesondere eine Pentosanase wie Xylanase (nützlich für die Teilhydrolyse von Pentosanen, die die Extensibilität des Teigs verbessern), Proteasen (nützlich für die Klebererweichung, insbesondere bei Verwendung von Hartweizenmehl), Proteindisulfidisomerase (beispielsweise eine Proteindisulfidisomerase, die in der WO95/00636 offenbart ist), Glycosyltransferase, Peroxidase (nützlich zur Verbesserung der Konsistenz des Teigs, Laccase oder Oxidase, zum Beispiel eine Aldoseoxidase, Glucoseoxidase, Pyranoseoxidase, Lipoxygenase oder L-Aminosäureoxidase (nützlich zur Verbesserung der Konsistenz des Teigs) sein.

Diese(s) gegebenenfalls weiter zugesetzte(n) Enzym bzw. Enzyme kann bzw. können separat oder zusammen mit dem erfindungsgemäßen Polypeptid mit Phospholipaseaktivität gegebenenfalls als Bestandteile von Back- bzw. Teighilfsmittel zugesetzt werden. Die Erfindung betrifft auch die Herstellung solcher Teige sowie die Herstellung entsprechender Backwaren aus diesen Teigen.

Die Erfindung betrifft ferner auch einen Prämix, zum Beispiel in Form einer Mehlzusammensetzung, zur Herstellung von Teig und/oder Backwaren aus Teig, wobei dieser Prämix erfindungsgemäße Polypeptide mit Phospholipaseaktivität umfasst.

Die erfindungsgemäßen Polypeptide mit Phospholipaseaktivität können auch als Zusatz zu Tierfutter verwendet werden. Die Zugabe von Phospholipasen zu Futter verbessert die Effizienz der Futterverwertung bei Tieren. Dadurch wird das Wachstum der Tiere, die mit solchem Futter ernährt werden, verbessert. Dabei kann eine erfindungsgemäße Phospholipase als solche oder als Futterkonzentrat zugesetzt werden. Ferner kann die Phospholipase auch über transgene Pflanzen dem Tierfutter zugesetzt werden, worin die Phospholipase durch heterologe Genexpression synthetisiert wurde. Verfahren zur Herstellung derartiger transgener Pflanzen sind in der EP0449376 offenbart.

Die erfindungsgemäßen Polypeptide mit Phospholipaseaktivität können auch bei dem Prozess des Scouring in der Textilrohstoffverarbeitung von z.B. Baumwollfasern eingesetzt werden um die weitere Bearbeitung der Fasern zu erleichtern. Die durch das Scouring erzielten Verbesserungen wirken sich auf das Verhalten beim Anfärben wie auch die weitere mechanische und enzymatische Verarbeitung der Faser wie auch des daraus hergestellten Gewebes aus.

Das Gen für die aus dem Mikroorganismus *Aspergillus fumigatus* isolierten Phospholipase wurde in dem Plasmid pPL3949-Topo2.5 unter der Hinterlegungsnummer DSM 22741 am 02.07.2009 bei der Deutsche Sammlung von Mikroorganismen und Zellkulturen GmbH (DSMZ), Inhoffenstraße 7B, D-38124 Braunschweig gemäß den Bedingungen des Budapester Vertrag hinterlegt.

Die Erfindung wird anhand der beigefügten Figuren näher erläutert. Es zeigen:

Figur 1: IEF Gel von gereinigter Phospholipase aus *Aspergillus fumigatus.*

Spur 1: Markerprotein aus dem Isoelectric Focusing Calibration Kit, pH 2,5-6,5 Spur 2-3: Die Phospholipasebande bei pI ca. 4.1 ist mit einem Pfeil gekennzeichnet.

Figur 2: T-Optimumskurve für die rekombinante Phospholipase exprimiert in *Trichoderma reesei* RH32664.

Figur 3: pH-Optimumskurve für die rekombinante Phospholipase exprimiert in *Trichoderma reesei* RH32664.

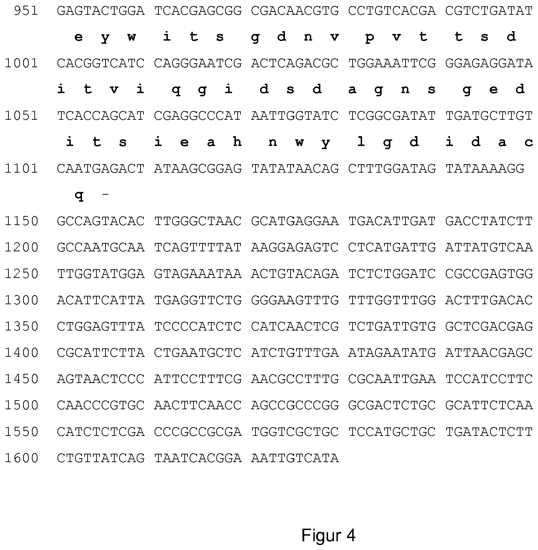

Figur 4: Nukleotidesequenz und daraus abgeleitete Aminosäuresequenz des chromosomalen Phospholipase Gens aus *Aspergillus fumigatus* RH3949. Die Introns sind in Kursivschrift und die Aminosäuresequenz in Fettschrift geschrieben. (SEQ ID NO: 1)

Figur 5: Die Nukleotidsequenz des chromosomalen Phospholipasegens aus *Aspergillus fumigatus* RH3949 (SEQ ID NO: 1).

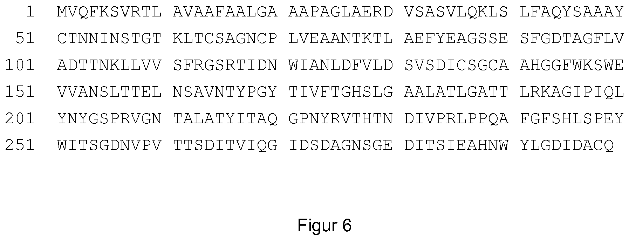

Figur 6: Die Aminosäuresequenz des Phospholipasegens aus *Aspergillus fumigatus* RH3949 (SEQ ID NO: 2)

Figur 7: Restriktionskarte des Vektors pPL3949-Topo2.5

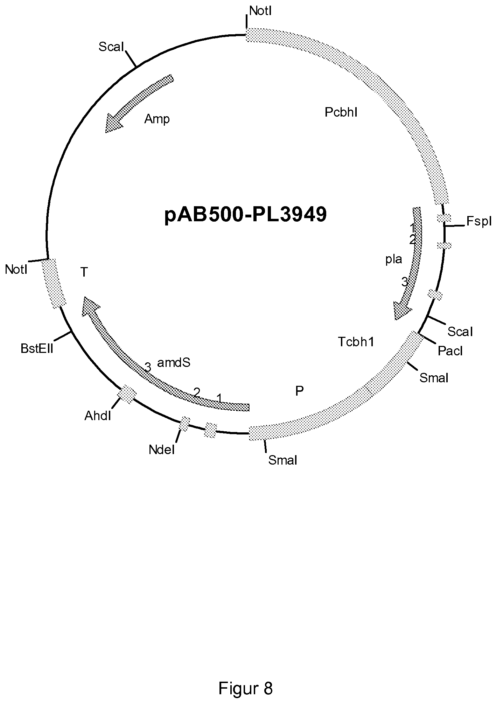

Figur 8: Restriktionskarte des Expressionsvektors pAB500-PL3949

**Referenzbeispiel 1**

**Bestimmung der Phospholipaseaktivität**

1 Phospholipase Unit (PLU) entspricht der Enzymmenge, die unter Standardbedingungen 1 $\mu$mol Fettsäure pro Minute aus Phosphatidylcholin freisetzt.

Reagenzien:

Substratemulsion:

1 g Epikuron 200 (gereinigtes Phosphatidylcholin aus Soja von Firma Lucas Meyer, jetzt erhältlich bei Cargill), 100 ml deionisiertes-Wasser und 5 ml 0,32 M CaCl$_2$-Lösung werden mit einem Ultra Turrax 2 min bei 24000 rpm homogenisiert.
Die Substratemulsion ist bei 4° - 8°C für 3 - 4 d haltbar.

Andere Lösungen:

0,32 MgCl$_2$-Lösung, frische 3,3 mM Zitronensäure - Monohydrat- Lösung, 10 mM KOH-Lösung, 1%-ige Triton X100 (Fa. Fluka) Lösung in VE-Wasser

Enzymlösung:

Die Enzympräparate werden in deionisiertem Wasser gelöst. Die Enzymkonzentration im Ansatz darf nicht über 2,5 U g$^{-1}$ liegen.

Durchführung:

Hauptwerte

10 ml Substratemulsion
10 ml 1%-ige Triton X100-Lösung
5 ml 3,3 mM Zitronensäure - Monohydrat- Lösung

wurden in einen 25-ml-Weithalserlenmeyerkolben pipettiert und 10 min auf 40°C temperiert. Der pH-Wert stellt sich auf ca. 3,3 - 3,5 ein.
Nach der Zugabe von 0,1 ml Enzymlösung wurde der Analysenansatz 10 min bei 40°C inkubiert. Nach Ablauf der Inkubationszeit wird mit 10mM KOH auf pH 10,0 titriert, wobei die ersten 5 ml KOH zügig (Dauer: ca.1 min) zugegeben werden. Der Verbrauch an KOH wird registriert.

Blindwerte

Die Enzymstammlösung wird 15 min bei 95°C erhitzt und somit deaktiviert. Nach dem Abkühlen auf Raumtemperatur erfolgt die weitere Behandlung wie bei den Hauptwerten.

Eine Inkubation der Blindwerte ist nicht notwendig.

Auswertung

$$PLU / g = \frac{\Delta V_{KOH} * c_{KOH} * 1000}{\Delta t * c_s * v}$$

| | |
|---|---|
| $V_{KOH}$ (ml) | Verbrauchdifferenz zwischen Blind und Hauptwert |
| $c_{KOH}$ (mol l$^{-1}$) | Konzentration der KOH Lösung |
| $\Delta t$ (min) | Inkubationszeit |
| cs (g ml$^{-1}$) | Konzentration der Probe |
| v (ml) | eingesetztes Volumen |

**Referenzbeispiel 2**

**Phospholipaseschnelltest bei pH 3,5**

Reagenzien:

Substratemulsion:

1 g Epikuron-200 werden mit 100 g Milli Q Wasser und 5 ml 0,32 M Calciumchloridlösung versetzt und mit dem Ultra-Turrax homogenisiert (ca. 1-2 min bei ca. 24000 rpm).
Für den Analysenansatz werden 10 ml dieser Substratemulsion mit 10 ml 1%-iger Triton X-100-Lösung und 5 ml 3,3 mM Citronensäure-Monohydrat-Lösung versetzt.
Freie Fettsäuren, Halbmikro-Test (Boehringer)
(Der Halbmikro-Test enthält die Reagenzien für das Reaktionsgemisch A, Reaktionsgemisch B und das N-Ethylmalei-nimid)

Durchführung:

5 $\mu$l verdünnte Enzymlösung werden in einer Mikrotiterplatte vorgelegt und mit 0,1 ml Substratemulsion versetzt. Die Substrat/Enzym-Ansätze werden 10 min bei 40°C im Wasserbad inkubiert. Anschließend werden 5 $\mu$l des Ansatzes in eine zweite Mikrotiterplatte zu 100 $\mu$l Reaktionsgemisch A pipettiert und 5 min bei 40°C im Wasserbad inkubiert. Nach Ablauf der Inkubationszeit werden 5 $\mu$l eines Gemisches im Verhältnis 1:1 von Reaktionsgemisch B und N-Ethylmaleinimid-Lösung zugegeben und erneut 5 min bei 40°C inkubiert.
Phospholipaseaktivität ist an einer Rotfärbung des Reaktionsansatzes zu erkennen.

**Referenzbeispiel 3**

**Bestimmung des Gehalts an freien Fettsäuren**

Reagenzien:

Ethanol 96%-ig, Toluol,
Ethanol und Toluol werden im Verhältnis 1:1 (v/v) gemischt.
0,1 N ethanolische KOH
1 % Phenolphthalein-Lösung in Ethanol

Durchführung:

Ca. 3g wasserfreies Öl werden in einem Erlenmeyerkolben auf 4 Kommastellen genau eingewogen, in 20 ml Ethanol-Toluol-Gemisch gelöst, mit 2 bis 3 Tropfen Phenolphthalein versetzt und mit 0,1 N KOH-Lösung bis zur bleibenden Rotfärbung titriert.

Auswertung:

Die Säurezahl ist ein Maß für den Gehalt an freien Fettsäuren. Die Säurezahl bezeichnet die Menge an Kaliumhydroxid in g, die zur Neutralisation von in 1kg Öl enthaltenen freien Fettsäuren notwendig ist. Die Säurezahl (SZ) wird nach folgender Gleichung berechnet:

$$SZ = \frac{a*N * 56,1}{E}$$

a      Verbrauch an KOH-Lösung in ml
N      Normalität von KOH
E      Fetteinwaage in g
56,1      Molare Masse von KOH in g/mol

Aus der Säurezahl lässt sich der prozentuale Gehalt an freien Fettsäuren (FFA) berechnen:

$$FFA\ (\%) = SZ\ *\ \frac{282 * 100}{56,1 * 1000}$$

282      Molare Masse der Ölsäure

**Referenzbeispiel 4**

**Lipase-Nachweis**

Der qualitative Lipase-Nachweis auf Olivenöl-Agar wird analog nach der Methode von Kouker und Jaeger durchgeführt (Applied Environ. Microbiol., 59: 211-213 (1987)).
Zur Detektion der Lipase-Aktivität wird aus Tributyrinagar (Fluka 91015) mit 1% Olivenöl hergestellte Agarplatten verwendet. Der pH-Wert stellt sich auf 5,5 ein.
Die Lipaseaktivität wird photometrisch mit emulgiertem p-Nitrophenylpalmitat (Sigma N2752) als Substrat in 0,5 M Citrat/Phosphatpuffer, pH 5,1 analog nach Winkler und Stuckmann (1979) durchgeführt (J. Bac., 138:663-670 (1979)).

**Beispiel 1**

**Gewinnung von Phospholipase aus *Aspergillus fumigatus Stamm RH3949***

*Aspergillus fumigatus* wurde in 200 ml Schüttelkolben, gefüllt mit 50 ml Medium bei 28°C, 200 rpm, über 5 d angezüchtet. Das Medium bestand aus 0,5% Epicuron 200 (Lucas Meyer), 0,5% Maisquellpulver, 0,2% $NH_4NO_3$, 100 mM $KH_2PO_4$ und 0,1% Triton X100. Der pH-Wert wurde vor dem Sterilisieren auf pH 6 eingestellt. Das Medium wurde mit einer Sporensuspension beimpft. Nach 5 Tagen wurde der Kulturüberstand von dem Mycel durch Filtration getrennt und die Phospholipaseaktivität in der Flüssigkeit gemessen.

**Beispiel 2**

**Reinigung der Phospholipase aus *Aspergillus fumigatus Stamm RH3949***

**Schritt 1:** Anionenaustauscher, Macro Prep Q

Zur Aufreinigung der Phospholipase wurde zuerst eine Trennung der Proteine am Anionenaustauscher Macro Prep Q durchgeführt.
Dazu wurde der aufkonzentrierte Kulturüberstand aus den Züchtungen nach Beispiel 1 mit vollentsalztem Wasser verdünnt, bis die Proteinlösung die gleiche Leitfähigkeit wie die Leitfähigkeit des Puffers A hatte. Anschließend wurde die Proteinprobe mit 1 M NaOH auf pH 7 eingestellt und auf die mit Puffer A equilibrierte Säule geladen. Nach dem Waschen der Säule mit Puffer A wurde die Phospholipase mit einem linear ansteigenden NaCl-Gradienten von 0-1M eluiert. Die Fraktionen mit Phospholipase-Aktivität wurden zusammengefasst und weiter gereinigt.

Puffer A : 5 mM $CaCl_2$ + 20 mM Tris-HCl, pH 7,0
Puffer B : 5 mM $CaCl_2$ + 20 mM Tris-HCl, pH 7,0 + 1M NaCl

**Schritt 2**: HIC, Phenyl Sepharose 6 Fast Flow low substitution

Die Proteinprobe mit Phospholipase Aktivität aus Schritt 1 wurde im Verhältnis 1:1 mit 3,4 M Ammoniumsulfat-Lösung gemischt und mit 1M NaOH-Lösung auf pH 7,0 eingestellt. Nach dem Auftragen der Probe auf die ebenfalls mit Puffer A equilibrierte Phenyl-Sepharose-Säule wurde die Phospholipase mit einem abnehmenden Ammoniumsulfat-Gradienten eluiert.

Puffer A: 5 mM $CaCl_2$ + 20 mM Tris-HCl, pH 7,0 + 1,7 M Ammoniumsulfat
Puffer B: 5 mM $CaCl_2$ + 20 mM Tris-HCl, pH 7,0

**Schritt 3:** Gelfiltration, Superose 12 HR 10/30

Als letzter Reinigungsschritt erfolgte eine Trennung der Proteine an einer Gelfiltrationsäule. Dazu wurde die Phospholipaseprobe aus Schritt 2 im Dialyseschlauch (Naturin Eiweiß-Saitling) gegen vollentsalzes Wasser für 1.5 h dialysiert and anschließend lyophilisiert. Das Lyophilisat wurde in 500 $\mu$l vollentsalztem Wasser aufgenommen. In zwei Läufen wurden jeweils 250 $\mu$l auf die Säule aufgetragen und mit dem Puffer A eluiert.
Puffer A: 5 mM $CaCl_2$ + 20 mM Tris-HCl, pH 7,0

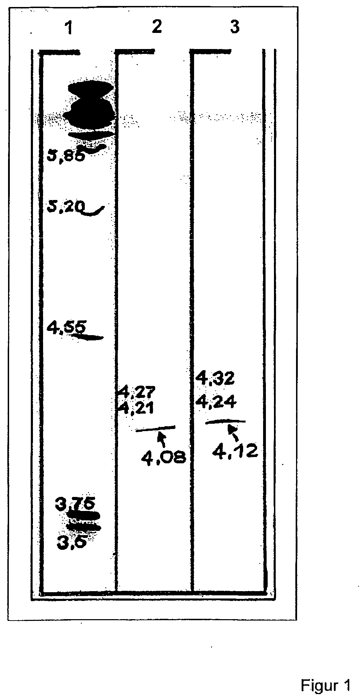

Die gereinigte Phospholipase wurde auf ein IEF Gel aufgetragen. Das Ergebnis ist in Figur 1 darstellt. Zur Identifikation wurden die Banden ausgeschnitten und entsprechend der beschriebenen Analysen-Methode auf Phospholipaseaktivität überprüft.

**Beispiel 3**

**N-Terminale Proteinsequenzierung**

Nach dem letzten Reinigungsschritt über die Superose wurde das gereinigte Protein auf einem Nativ-Gel getrennt. Die Proteinbanden mit Phospholipaseaktivität wurden ausgeschnitten und erneut auf ein SDS-Gel aufgetragen, um das Molekulargewicht zu bestimmen. Zur N-terminalen Aminosäurebestimmung wurden die Proteinbanden aus dem Nativ-Gel auf eine PVDF-Membran (Fluotrans Transfer Membrane, Pall) transferiert und nach der Coomassie-Färbung die N-terminalen Aminosäuresequenzen in einem Aminosäure-Sequenzer (Applied Biosystems Model 470A) bestimmt. Sie lauten:

[1]DVSAS VLQKL SLFAQ Y[16] (SEQ ID NO: 3)

Der Sequenzvergleich zeigt, daß die N-terminale Aminosäuresequenz des Phospholipase Gens eine hohe Sequenzverwandschaft mit dem Gen der extrazellulären Lipase aus dem *Aspergillus fumigatus* Stamm Af293 (GenBank EAL86100) aufweist.

**Beispiel 4**

**Klonierung des chromosomalen Phospholipasegens aus dem *Aspergillus fumigatus* Stamm RH3949 mittels Polymeraseketten-Reaktion (PCR)**

Aus den Daten der chromosomalen DNA-Sequenz der *Aspergillus fumigatus*-Lipase wurden verschiedene Oligoprimer für die Amplifizierung der Phospholipase-DNA abgeleitet. Die chromosomale DNA Präparation wurde nach einer modifizierten Vorschrift von Hynes, M.J et al., (1983) Mol. Cell. Biol. 3, 1430-1439, durchgeführt. Die Amplifizierung eines Phospholipasegens wurde mit der PCR-Methode durchgeführt. Die PCR-Produkte wurden in pCR2.1-TOPO-Plasmid kloniert und sequenziert. Dabei zeigt sich, dass das Primerpaar N2-3948 und 3949-Apal zu dem Gen mit der erfindungsgemäßen Phospholipase-DNA führt.
N2-3948 5'- AGAGTCTGCC TATATTCTCT CTGAAAGG -3' (SEQ ID NO. 4) 3949-Apal5'-TATGACAATTTCCGTGAT-TACTG-3' (SEQ ID NO: 5)

Der Reaktionsansatz von 100 $\mu$l enthielt: 10$\mu$l 10 x Puffer (200 mM Tris/HCl, pH 8,4, 500 mM KCl), 3$\mu$l 50mM MgCl$_2$, 2 $\mu$l 10 mM dNTP, jeweils 50 pMol Oligoprimer (N2-3948 und 3949-Apal), ca. 10 ng chromosomale DNA, 5U Taq DNA-Polymerase (Invitrogen). Der Ansatz wurde für die Denaturierung bei 95°C / 5 min, 45 Zyklen (95°C / 1 min, 45°C / 1 min, 72°C / 1 min) und anschließend die Extention bei 72°C / 10 min durchgeführt.

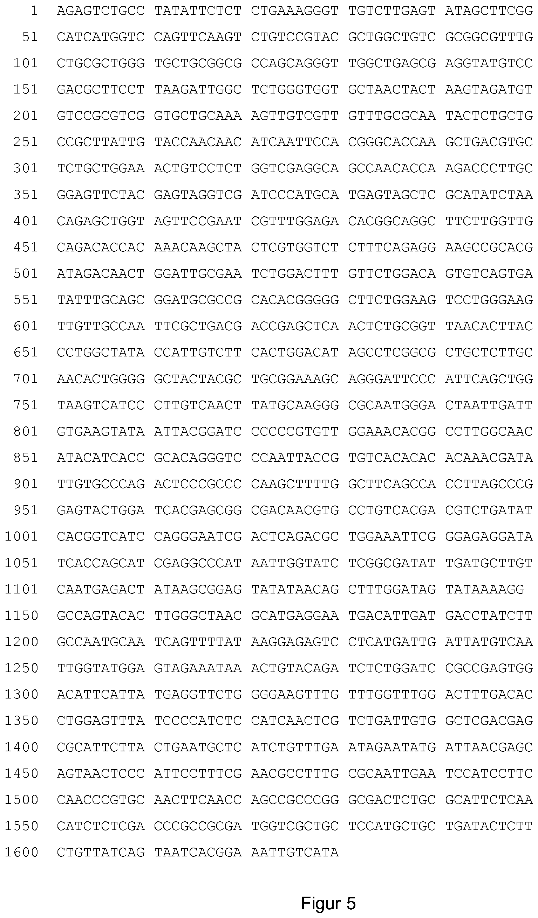

Die PCR-Produkte wurden über Qiaquick-Säule gereinigt und in pCR2.1-TOPO-Plasmid kloniert. Ein Transformant enthielt nach der Sequenzierung die erfindungsgemäße Phospholipase DNA Sequenz (Figur 5, SEQ ID NO: 1) und wurde als pPL3949-Topo2.5 bezeichnet (Figur 7).

Der offene Leserahmen, der für die Phospholipase kodiert, umfasst 1052 Nukleotide enthaltend 299 Aminosäuren. Das Phospholipase Gen enthält 3 Introns.

Die abgeleitete N-terminale Aminosäuresequenz stimmt mit den aus der Proteinsequenzierung ermittelten Peptidsequenzen überein (Beispiel 3, SEQ ID NO: 3).

Das abgeleitete Molekulargewicht von ca. 28,6 kDa entspricht den ca. 29 kDa, die durch SDS-PAGE ermittelt wurden (Beispiel 8).

Die Ermittlung der Signalsequenz wurde mit einem Computerprogramm (PSORT) von Nakai und Kanehisa (1992, Genomics 14, 897-911) durchgeführt. Daraufhin weist das Phospholipase-Gen eine Signalsequenz von 21 Aminosäuren und ein Propeptid von 8 Aminosäuren auf.

**Beispiel 5**

**Konstruktion des Expressionsvektors pAB500-PL3949**

In dem Expressionsvektor pAB500-PL3949 steht das Phospholipase Gen unter der Kontrolle des *T. reesei* cbhl Promotors und cbhl Terminators.

Zur Konstruktion des Plasmids pAB500-PL3949 wurde das für Phospholipase kodierende Gen aus dem Plasmid pPL3949.Topo2.5 mittels PCR amplifiziert.

Das PCR-Produkt wurde mit den Enzymen AvrII/PacI hydrolysiert und anschließend in die SpeI und PacI Spaltstellen nach dem *T. reesei* cbh1 Promotor in dem Plasmid pAB500 inseriert. Das erhaltene Plasmid hat die Bezeichnung pAB500-PL3949.

Die Konstruktion des Plasmids pAB500 erfolgte durch folgende Schritte:

Aus dem Plasmid pALK487 (WO94/28117) wurde durch Einfügen weiterer Schnittstellen (SpeI und PacI in die SacII-Stelle zwischen dem cbhl-Promotor und cbhl-Terminator) das Plasmid pAB487 hergestellt. Die SpeI-PacI-Schnittstellen werden für die direkte Klonierung des Phospholipasegens verwendet.

Das amdS Gen einschließlich seines Promotors und seines Terminators wurde aus dem Plasmid p3SR2 (GenBank 16371) mittels der PCR amplifiziert. Das PCR-Produkt wurde mit Ascl und NruI geschnitten und in die Ascl / StuI-Spaltstelle von pAB487 inseriert, wodurch das Plasmid pAB500 erhalten wurde.

**Beispiel 6**

**Transformation von T. *reesei***

Die zur Transformation und zur Handhabung von T. *reesei* verwendeten Techniken waren gemäß Penttilä et al. (1987, Gene 61: 155-164).

*T. reesei* RH32439 wurde mit der aus dem Plasmid pAB500-PL3949 isolierten linearisierten Expressionskassette transformiert.

Die Transformanten wurden selektiert und durch Einzelsporenisolierung gereinigt. Von allen Transformanten wurden diejenigen mit der höchsten Sekretionsleistung ausgewählt und in Beispiel 7 zur Herstellung von Enzymmaterial weiter verwendet.

**Beispiel 7**

**Herstellung von Enzymlösungen durch Fermentation in Schüttelkolben**

Transformanten, die die Expressionskassette aus dem Beispiel 6 tragen, wurden in Schüttelkolben auf Cellulase-induzierendem Medium kultiviert. Die nach 6-tägiger Züchtung erhaltenen Kulturfiltrate wurden für die Charakterisierung des Enzymes (Beispiel 8) und für die Analyse der Ölentschleimung (Beispiel 9) verwendet.

**Beispiel 8**

**Charakterisierung der rekombinanten Phospholipasen**

Die Molekulargewichtsbestimmung durch SDS-Polyacrylamidgelelektrophorese (SDS-PAGE) ergab ein Molekulargewicht von ca. 29 kDa für die erfindungsgemäße Phospholipase.

Die N-terminale Sequenzierung der rekombinanten Phospholipase wurde durch die Firma Chromatec (Germany) durchgeführt. Die durch die Sequenzierung erhaltene Aminosäuresequenz stimmt mit der N-terminalen Sequenz der Phospholipase (Beispiel 3) überein.

Die Identifizierung des rekombinanten Proteins mit MALDI-MS wurde von der Firma Protagen (Germany) durchgeführt. Das Ergebnis bestätigt die Richtigkeit der Proteinsequenz der erfindungsgemäßen Phospholipase.

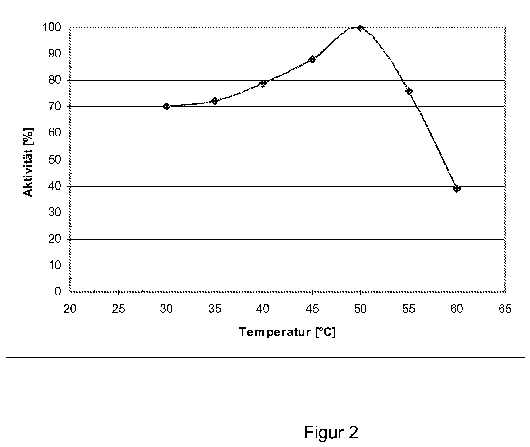

Die Temperaturabhängigkeit der Enzymaktivität wurde mit der Bestimmungsmethode wie oben beschrieben bei verschiedenen Temperaturen ermittelt. Das Temperaturoptimum der Phospholipase liegt bei 50° C (vgl. Figur 2).

Der optimale pH-Wert der Enzym-Aktivität wurde mit der Bestimmungsmethode wie oben beschrieben bei verschiedenen pH-Werten ermittelt. Der pH-Wert im Reaktionsansatz wurde dazu mit Hilfe von Zitronensäure eingestellt.

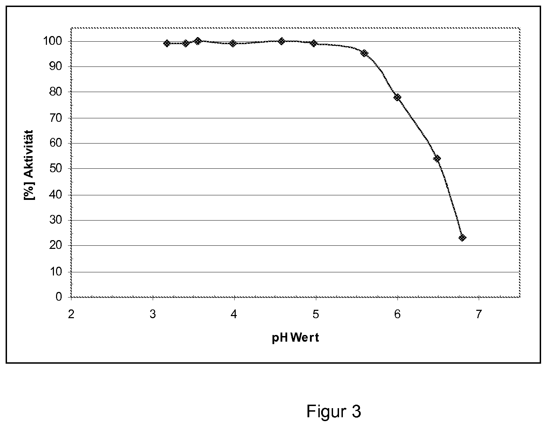

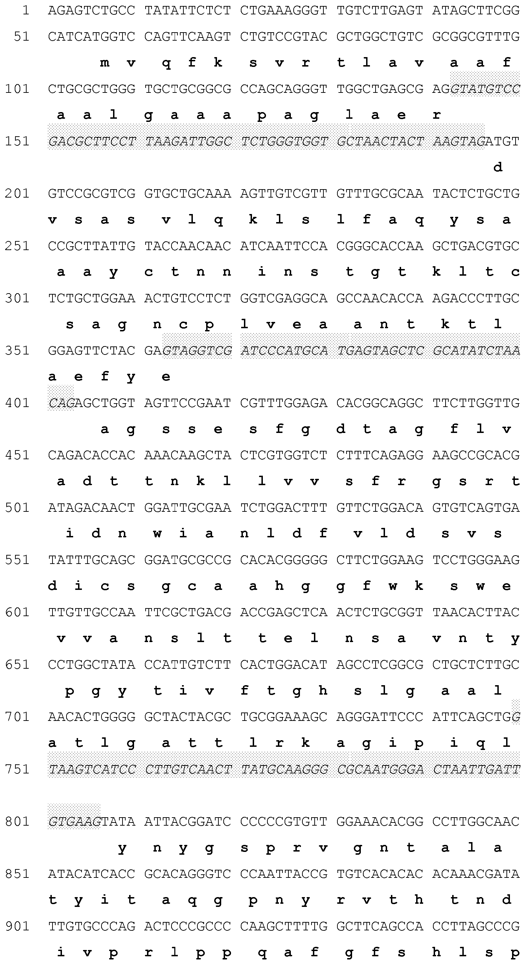

Das Enzym ist in einem weiten pH-Bereich von pH 3 - 5 aktiv (vgl. Figur 3).

Die Lipaseaktivität wurde mit der oben beschrieben Bestimmungsmethode ermittelt. Die erfindungsgemäße *A. fumigatus* Phospholipase weist eine sehr geringe Lipase-Aktivität auf. Das Verhältnis Phospholipase- zu Lipase-Aktivität wurde als 7.480:1 ($\pm$ 10% experimentelle Schwankungsbreite) ermittelt.

**Beispiel 9**

**Entschleimung von Öl mit Enzym aus Kulturüberständen von rekombinanten *Trichoderma reseei* Stämmen mit dem Gen aus *A. fumigatus* RH 3949**

In einem 400ml Becherglas wurden 200 g Speiseöl (Sojaöl 1 mit 163,6 ppm Phosphorgehalt; Rapsöl mit 161,6 ppm Phosphorgehalt, Sojaöl 2 mit 592.8 ppm Phosphorgehalt und Sojaöl 3 mit 81,1 ppm Phosphorgehalt) mit 0,42 ml einer 46%igen Citronensäure Lösung und Wasser versetzt. Dabei soll der Gesamtwassergehalt von 2 % nicht überschritten werden. Der pH-Wert der Reaktionsmischung wurde durch Zugabe von 7%iger NaOH Lösung (0,6 ml) auf pH 4,0 eingestellt. Nach Zugabe der erfindungsgemäßen Enzymlösung (50 U) wurde der Reaktionsansatz mit Hilfe eines Ultra Turrax für 2 Min. bei 24000 rpm gemischt. Anschließend wurde die Mischung in einen Dreihalsrundkolben überführt und unter leichtem Rühren (200U/min) bei 55°C bzw. 60°C inkubiert.

Die Probenentnahme von 20 ml erfolgte alle 120 min nach Zugabe der Enzymlösung. Die Proben wurden für 5 min bei 4300 x g zentrifugiert und der Phospholipidgehalt, ausgedrückt in ppm Phosphor, wurde im Öl nach Veraschung bei 850°C unter Zugabe von Magnesiumoxid, als Phosphomolybdatkomplex photomoetrisch bei 830 nm bestimmt.

Der rekombinante *Trichoderma reesei* Stamm, der das erfindungsgemäße Plasmid pAB500-PL3949 enthält, trägt die Bezeichnung RH32664.

Die Ergebnisse der Entschleimung von Speiseöl bei 55°C bzw. 60°C mit aus rekombinanten Stamm hergestellter Phospholipase aus *Aspergillus fumigatus* RH 3949, zeigt Tabelle 2.

Die Ergebnisse zeigen eine deutliche Entschleimungswirkung durch das erfindungsgemäße Enzym im Vergleich zur Wasserentschleimung mit Zitronensäure. Bereits nach 4 Stunden beträgt der Restphosphorgehalt weniger als 10 ppm.

Tab.: 2: Entschleimung von Sojaöl und Rapsöl mit erfindungsgemäßen Phospholipase-Enzymen (250 U je kg/ Speiseöl) aus Kulturüberständen von rekombinantem *Trichoderma reesei* Stamm mit 2 % Wassergehalt bei pH 4,0

| Probebezeichnung | | Phosphorgehalt (ppm) | | |
|---|---|---|---|---|
| | Zeit (min) | Temp (°C) | | |
| | | 55 | 60 | |
| Citronensäure | 240 | 109,7 | 15,5 | 14,9 |
| | 360 | 117,7 | 13,4 | 15,3 |
| RH32664 (Phospholipase) | 240 | 8,4 | 4,1 | 4,6 |
| | 360 | 7,8 | 2,4 | 2,0 |
| | | | | |
| Sojaöl 1 | unbehandelt | 163,3 | | |

(fortgesetzt)

| Probebezeichnung | | Phosphorgehalt (ppm) | | |
|---|---|---|---|---|
| | Zeit (min) | Temp (°C) | | |
| Rapsöl | unbehandelt | | 161,6 | |
| Sojaöl 2 | unbehandelt | | | 592,8 |

**Beispiel 10**

**Gehalt an freien Fettsäuren bei dem Öl-Entschleimungsprozess**

Die Bestimmung des Gehalts an freier Fettsäure bei dem Ölentschleimungsprozess wurde wie in Referenzbeispiel 3 beschrieben durchgeführt. Das Speiseöl wurde mit Phospholipase wie in dem Beispiel 9 gemischt und 6 Stunden bei 57°C inkubiert. Die enzymatische Hydrolyse bei Phospholipiden wurde durch Phospholipase hervorgerufen, die die Fettsäure abspalten. Zum Vergleich wurde die gleiche Analyse mit reinen Speiseölen als Blindwert durchgeführt.

Im Vergleich zum Blindwert (BW), steigt der Gehalt an freier Fettsäure (FFA) in den Proben nur geringfügig an und die Differenz ($\Delta_{FFA}$) liegt bei 0,18% nach der Behandlung von Speiseöl mit Phospholipase bei einer Temperatur von 57°C (Tab. 3).

Tab. 3: Gehalt an freien Fettsäuren nach der Behandlung des Öls mit Phospholipase.

| Probebezeichnung | Zeit (min) | Phosphorgehalt (ppm) | FFA (%) | $\Delta_{FFA}$ (%) |
|---|---|---|---|---|
| Citronensäure | 240<br>360 | 37,2<br>42,4 | <br>0,09 | |
| | | | | |
| RH32664<br>(Phospholipase) | 240<br>360 | 2,0<br>3,0 | <br>0,27 | <br>0,18 |
| | | | | |
| Sojaöl.3, unbehandelt | | 81,1 | | |

**Patentansprüche**

1. Nukleinsäuremolekül mit einer DNA-Sequenz, die ein Polypeptid mit Phospholipaseaktivität im Wesentlichen ohne Lipaseaktivität, photomerisch bestimmt durch Hydrolyse von p-Nitrophenylpalmitat, kodiert, **dadurch gekennzeichnet, dass** die DNA-Sequenz ausgewählt ist aus

   a) DNA-Sequenzen, die eine Nukleotidsequenz gemäß SEQ ID NO: 1 umfassen,
   b) DNA-Sequenzen, die die kodierende Sequenz gemäß SEQ ID NO: 1 umfassen,
   c) DNA-Sequenzen, die die Proteinsequenz gemäß SEQ ID NO: 2 kodieren,
   d) DNA-Sequenzen, die von dem Plasmid pPL3940-Topo2.5 mit der Restriktionskarte gemäß Figur 7 und hinterlegt unter der Hinterlegungsnummer DSM 22741 kodiert werden,
   e) DNA-Sequenzen, die aufgrund der Degeneriertheit des genetischen Codes mit den DNA-Sequenzen gemäß a), b), c) oder d) verwandt sind, und
   f) komplementären Strängen zu den Sequenzen gemäß a) bis e),

   wobei die DNA-Sequenz bevorzugt aus *Aspergillus* und noch bevorzugter aus *Aspergillus fumigatus* abgeleitet ist.

2. Nukleinsäuremolekül, umfassend ein Analogon einer der Sequenzen gemäß Anspruch 1, **dadurch gekennzeichnet, dass** die Sequenz ein Polypeptid mit Phospholipaseaktivität im Wesentlichen ohne Lipaseaktivität, photomerisch bestimmt durch Hydrolyse von p-Nitrophenylpalmitat, kodiert und

   a) mindestens 88% Identität zu einer der Sequenzen gemäß b), c), e) und f) aufweist, wobei die Sequenz erhältlich ist durch Substitution, Deletion, Insertion, Addition oder Mutation einer oder mehrerer Nukleinsäuren der DNA-Sequenz gemäß SEQ ID NO: 1 oder
   b) ein komplementärer Strang zu den Sequenzen gemäß a) ist, wobei die Sequenz erhältlich ist durch Substitution, Deletion, Insertion, Addition oder Mutation einer oder mehrerer Nukleinsäuren der DNA-Sequenz gemäß SEQ ID NO: 1.

3. Expressionskonstrukt umfassend eine Sequenz nach einem der Ansprüche 1 bis 2 in funktioneller Verknüpfung mit einer oder mehreren Sequenz(en) zur Steuerung der Expression des Polypeptids mit Phospholipaseaktivität in einer geeigneten Wirtszelle, wobei die Sequenz zur Steuerung der Expression des Polypeptids bevorzugt ein Promotor, ausgewählt aus dem Glucoamylase- oder α-Amylase-Promotor der Gattung *Aspergillus,* dem Cellulase (Cellobiohydrolase-)-Promotor der Gattung *Trichoderma,* einem Promotor für ein Enzym im glykolytischen Stoffwechselweg wie Phosphoglyceratkinase oder Glycerinaldehyd-3-phosphatdehydrogenase, dem Xylanasepromotor oder dem Enolase-Promotor ist und gegebenenfalls umfassend weiterhin eine sekretorische Leadersequenz.

4. Rekombinante Wirtszelle, **dadurch gekennzeichnet, dass** sie mit einem Expressionskonstrukt nach Anspruch 3 transformiert worden ist.

5. Rekombinante Wirtszelle nach Anspruch 4, **dadurch gekennzeichnet, dass** sie von einer Pilzzelle der Gattung *Aspergillus, Rhizopus, Trichoderma, Neurospora, Mu*cor oder *Penicillium* oder einer Hefezelle der Gattung *Kluyveromyces, Saccharomyces, Schizosaccharomyces, Trichosporon, Schwanniomyces, Hansenula* oder *Pichia* abgeleitet ist.

6. Plasmid pPL3949-Topo2.5 mit der Restriktionskarte gemäß Figur 7 und hinterlegt unter der Hinterlegungsnummer DSM 22741.

7. Polypeptid mit Phospholipaseaktivität im Wesentlichen ohne Lipaseaktivität ausgewählt aus

   a) einem Polypeptid, das von dem kodierenden Teil einer DNA-Sequenz nach einem der Ansprüche 1 bis 2 kodiert wird,

b) einem Polypeptid mit einer Sequenz, die mindestens 83% Identität zu den Aminosäuren 1 bis 299 von SEQ ID NO: 2 aufweist,

c) einem Polypeptid, das von einer Nukleinsäuresequenz kodiert wird, die unter stringenten Bedingungen hybridisiert mit

(i) den Nukleotiden 55 bis 1106 von SEQ ID NO: 1,
(ii) der in den Nukleotiden 55 bis 1106 von SEQ ID NO: 1 enthaltenen cDNA-Sequenz, oder
(iii) einem Komplementärstrang von (i) oder (ii).

8. Polypeptid mit Phospholipaseaktivität nach Anspruch 7, **dadurch gekennzeichnet, dass** es

- ein Molekulargewicht im Bereich von 28 bis 30 kDa besitzt
- mindestens eine der zwei Fettsäuren aus Lecithin hydrolysieren kann,
- im Wesentlichen keine Lipaseaktivität aufweist,
- eine hohe Temperaturbeständigkeit besitzt und
- aus einem Organismus der Gattung *Aspergillus* isolierbar ist.

9. Polypeptid nach Anspruch 8, **dadurch gekennzeichnet, dass** es aus *Aspergillus* fumigatus isolierbar ist.

10. Polypeptid nach Anspruch 9, **dadurch gekennzeichnet, dass** es immunologisch mit einem gegen die Sequenz SEQ ID NO: 2 gerichteten Antikörper reaktiv ist.

11. Polypeptid nach einem der Ansprüche 8 bis 10, **dadurch gekennzeichnet, dass** es die Sequenz gemäß SEQ ID NO: 2 umfasst.

12. Phospholipasezusammensetzung, **dadurch gekennzeichnet, dass** sie ein Polypeptid nach einem der Ansprüche 7 bis 11 zusammen mit Hilfsstoffen umfasst und dass sie gegebenenfalls weiterhin ein oder mehrere Enzyme für Nahrungs- oder Futtermittel umfasst.

13. Verwendung eines Polypeptids nach einem der Ansprüche 7 bis 11 oder einer Phospholipasezusammensetzung nach Anspruch 12 zur Entschleimung von pflanzlichem Öl, zur Herstellung von Teig und/oder Backwaren, zur Herstellung von Milchprodukten, als Zusatz zu Tierfutter oder zur Bearbeitung von Textilrohstoffen.

14. Verfahren zur Produktion eines Polypeptids mit Phospholipaseaktivität nach einem der Ansprüche 7 bis 11, **dadurch gekennzeichnet, dass** man eine Wirtszelle nach einem der Ansprüche 4 oder 5 unter Bedingungen, die die Expression des Polypeptids fördern, züchtet und anschließend das Polypeptid gewinnt.

15. Verfahren zur Entschleimung von pflanzlichem Öl, **dadurch gekennzeichnet, dass** man

a) das zu behandelnde pflanzliche Öl gegebenenfalls vorbehandelt,
b) dem so gegebenenfalls vorbehandelten Öl eine wässrige ein Polypeptid nach einem der Ansprüche 7 bis 11 enthaltende Lösung oder eine Phospholipasezusammensetzung nach Anspruch 12 zusetzt,
c) den Reaktionsansatz nach b) 1 bis 12 h auf eine Temperatur zwischen 30° und 70°C erhitzt, und
d) die wässrige und ölige Phase voneinander trennt.

## Claims

1. A nucleic acid molecule with a DNA sequence which codes for a polypeptide having phospholipase activity essentially without lipase activity photometrically determined by means of hydrolysis of p-nitrophenylpalmitate **characterized in that** the DNA sequence is selected from

a) DNA sequences that comprise a nucleotide sequence according to SEQ ID NO: 1,
b) DNA sequences that comprise the coding sequence according to SEQ ID NO: 1,
c) DNA sequences that code for the protein sequence according to SEQ ID NO: 2,
d) DNA sequences that are coded for by the plasmid pPL3940-Topo2.5 with the restriction map according to figure 7, which is deposited under accession number DSM 22741,
e) DNA sequences that are related to the DNA sequences according to a), b), c) or d) due to the degeneration of the genetic code, and

f) complementary strands to the sequences according to a) to e), wherein the DNA sequence is preferably derived from *Aspergillus* and more preferably from *Aspergillus fumigatus.*

2. A nucleic acid molecule which comprises an analogue of one of the sequences according to claim 1 **characterized in that** the sequence codes for a polypeptide having phospholipase activity essentially without lipase activity photometrically determined by means of hydrolysis of p-nitrophenylpalmitate and

a) has at least 88% identity with one of the sequences according to b), c), e) and f), wherein the sequence may be obtained by substitution, deletion, insertion, addition or mutation of one or more nucleic acid(s) of the DNA sequence according to SEQ ID NO: 1, or

b) is a complementary strand to the sequences according to a), wherein the sequence may be obtained by substitution, deletion, insertion, addition or mutation of one or more nucleic acid(s) of the DNA sequence according to SEQ ID NO: 1.

3. An expression construct which comprises a sequence according to one of claims 1 to 2 in operable linkage with one or more sequence(s) used for directing the expression of the polypeptide having phospholipase activity in an appropriate host cell, wherein the sequence used for directing the expression of the polypeptide is preferably a promoter selected from the glucoamylase promoter or the α-amylase promoter of the genus *Aspergillus,* the cellulase (cellobiohydrolase) promoter of the genus *Trichoderma,* a promoter for an enzyme in the glycolytic metabolic pathway such as phosphoglycerate kinase or glycerol aldehyde-3-phosphate dehydrogenase, the xylanase promoter or the enolase promoter, and which optionally further comprises a secretory leader sequence.

4. A recombinant host cell **characterized in that** it was transformed by means of an expression construct according to claim 3.

5. The recombinant host cell according to claim 4 **characterized in that** it is derived from a fungus cell of the genus *Aspergillus, Rhizopus, Trichoderma, Neurospora, Mucor* or *Penicillium* or from a yeast cell of the genus *Kluyveromyces, Saccharomyces, Schizosaccharomyces, Trichosporon, Schwanniomyces, Hansenula* or *Pichia.*

6. The pPL3949-Topo2.5 plasmid with the restriction map according to Figure 7 which is deposited under accession number DSM 22741.

7. A polypeptide having phospholipase activity essentially without lipase activity selected from

a) a polypeptide which is coded for by the coding part of a DNA sequence according to one of claims 1 to 2,

b) a polypeptide having a sequence that has at least 83% identity with the amino acids 1 to 299 of SEQ ID NO: 2,

c) a polypeptide which is coded for by a nucleic acid sequence which hybridizes under stringent conditions with

(i) nucleotides 55 to 1106 of SEQ ID NO: 1,

(ii) the cDNA sequence contained in nucleotides 55 to 1106 of SEQ ID NO: 1,

(iii) a complementary strand of (i) or (ii).

8. A polypeptide having phospholipase activity according to claim 7 **characterized in that** it

- has a molecular weight in the range of 28 to 30 kDa
- may hydrolyze at least one of the two fatty acids of lecithin
- essentially shows no lipase activity
- has a high temperature resistance
- may be isolated from an organism of the genus *Aspergillus.*

9. The polypeptide according to claim 8 **characterized in that** it may be isolated from *Aspergillus* fumigatus.

10. The polypeptide according to claim 9 **characterized in that** it is immunologically reactive with an antibody directed against the sequence SEQ ID NO: 2.

11. The polypeptide according to any one of claims 8 to 10 **characterized in that** it comprises the sequence according to SEQ ID NO: 2.

**12.** A phospholipase composition **characterized in that** it comprises a polypeptide according to any one of claims 7 to 11 together with additives and that it optionally further comprises one or more enzyme(s) for food or animal feed.

**13.** The use of a polypeptide according to any one of claims 7 to 11 or a phospholipase composition according to claim 12 for the degumming of vegetable oil, for the preparation of dough and/or bakery products, for the preparation of dairy products, as additive to animal feed or for the processing of textile raw materials.

**14.** The process for the production of a polypeptide having phospholipase activity according to any one of claims 7 to 11 **characterized in that** a host cell according to any one of claims 4 or 5 is grown under conditions which support the expression of the polypeptide and subsequently the polypeptide is extracted.

**15.** The process for the degumming of vegetable oil **characterized in that**

a) the vegetable oil to be treated is optionally pre-treated,
b) an aqueous solution containing a polypeptide according to any one of claims 7 to 11 or a phospholipase composition according to claim 12 is added to the thus optionally pre-treated oil,
c) the reaction batch according to b) is heated 1 to 12 h to a temperature of between 30°C and 70°C and
d) the aqueous and oily phases are separated.

**Revendications**

**1.** Molécule d'acide nucléique avec une séquence d'ADN qui code pour un polypeptide avec une activité de phospholipase sensiblement sans activité de lipase déterminée d'un point de vue photomérique par hydrolyse du p-nitrophénylpalmitate, **caractérisée en ce que** la séquence d'ADN est choisie parmi

a) des séquences d'ADN qui comprennent une séquence nucléotidique selon SEQ ID N° : 1,
b) des séquences d'ADN qui comprennent la séquence codante selon SEQ ID N° : 1,
c) des séquences d'ADN qui codent pour la séquence protéique selon SEQ ID N° : 2,
d) des séquences d'ADN qui sont codées par le plasmide pPL3940-Topo2.5 avec la carte de restriction selon la figure 7 et enregistrées sous le numéro d'enregistrement DSM 22741,
e) des séquences d'ADN qui sont apparentées aux séquences d'ADN selon les points a), b), c) ou d) en raison de la dégénérescence du code génétique, et
f) des brins complémentaires aux séquences selon les points a) à e),

dans laquelle la séquence d'ADN est dérivée de préférence d'*Aspergillus* et de façon davantage préférée, d'*Aspergillus fumigatus.*

**2.** Molécule d'acide nucléique, comprenant un analogue d'une des séquences selon la revendication 1, **caractérisée en ce que** la séquence code pour un polypeptide avec une activité de phospholipase sensiblement sans activité de lipase déterminée d'un point de vue photomérique par hydrolyse du p-nitrophénylpalmitate, et

a) présente une identité d'au moins 88 % avec l'une des séquences selon les points b), c), e) et f), dans laquelle la séquence peut être obtenue par substitution, délétion, insertion, addition ou mutation d'un ou plusieurs acides nucléiques de la séquence d'ADN selon SEQ ID N° : 1 ou
b) est un brin complémentaire aux séquences selon le point a), dans laquelle la séquence peut être obtenue par substitution, délétion, insertion, addition ou mutation d'un ou plusieurs acides nucléiques de la séquence d'ADN selon SEQ ID N° : 1.

**3.** Construction d'expression comprenant une séquence selon l'une des revendications 1 à 2 en liaison fonctionnelle avec une ou plusieurs séquence(s) pour la commande de l'expression du polypeptide avec une activité de phospholipase dans une cellule hôte appropriée, dans laquelle la séquence de commande de l'expression du polypeptide est de préférence un promoteur choisi parmi le promoteur glucoamylase ou α-amylase du genre *Aspergillus,* le promoteur de la cellulase (cellobiohydrolase) du genre *Trichoderma,* un promoteur pour une enzyme dans le métabolisme glycolytique tel que la phosphoglycérate-kinase ou la glycérinhaldéhyde-3-phosphate-déshydrogénase, le promoteur xylanase ou le promoteur énolase et comprenant en outre éventuellement une séquence leader sécrétrice.

**4.** Cellule hôte recombinante, **caractérisée en ce qu'**elle a été transformée avec une construction d'expression selon la revendication 3.

**5.** Cellule hôte recombinante selon la revendication 4, **caractérisée en ce qu'**elle est dérivée d'une cellule de champignon du genre *Aspergillus, Rhizopus, Trichoderma, Neurospora, Mucor* ou *Penicillium* ou d'une cellule de levure du genre *Kluyveromyces, Saccharomyces, Schizosaccharomyces, Trichosporon, Schwanniomyces, Hansenula* ou *Pichia.*

**6.** Plasmide pPL3949-Topo2.5 avec la carte de restriction selon la figure 7 et enregistré sous le numéro d'enregistrement DSM 22741.

**7.** Polypeptide avec une activité de phospholipase sensiblement sans activité de lipase, choisi parmi

a) un polypeptide qui est codé par la partie codante d'une séquence d'ADN selon l'une des revendications 1 à 2,
b) un polypeptide avec une séquence qui présente une identité d'au moins 83 % avec les acides aminés 1 à 299 de SEQ ID N° : 2,
c) un polypeptide qui est codé par une séquence d'acide nucléique qui s'hybride dans des conditions stringentes avec

(i) les nucléotides 55 à 1106 de SEQ ID N° : 1,
(ii) la séquence d'ADNc contenue dans les nucléotides 55 à 1106 de SEQ ID N° : 1, ou
(iii) un brin complémentaire de (i) ou (ii).

**8.** Polypeptide avec une activité de phospholipase selon la revendication 7, **caractérisé en ce qu'**il

- possède un poids moléculaire dans la plage de 28 à 30 kDa
- peut hydrolyser au moins l'un des deux acides gras de la lécithine
- ne présente sensiblement pas d'activité de lipase,
- possède une résistance élevée à la température et
- peut être isolé d'un organisme du genre *Aspergillus.*

**9.** Polypeptide selon la revendication 8, **caractérisé en ce qu'**il peut être isolé *d'Aspergillus fumigatus.*

**10.** Polypeptide selon la revendication 9, **caractérisé en ce qu'**il réagit immunologiquement avec un anticorps dirigé contre la séquence SEQ ID N° : 2.

**11.** Polypeptide selon l'une des revendications 8 à 10, **caractérisé en ce qu'**il comprend la séquence selon SEQ ID N° : 2.

**12.** Composition de phospholipase, **caractérisée en ce qu'**elle comprend un polypeptide selon l'une des revendications 7 à 11 conjointement avec des adjuvants et qu'elle comprend en outre éventuellement une ou plusieurs enzymes pour produits alimentaires ou aliments pour animaux.

**13.** Utilisation d'un polypeptide selon l'une des revendications 7 à 11 ou d'une composition de phospholipase selon la revendication 12 pour la démucilagination d'huile végétale, pour la fabrication de pâte et/ou de produits de boulangerie, pour la fabrication de produits laitiers, comme additif aux aliments pour animaux ou pour la transformation de matières premières textiles.

**14.** Procédé de production d'un polypeptide avec une activité de phospholipase selon l'une des revendications 7 à 11, **caractérisé en ce qu'**une cellule hôte est cultivée selon l'une des revendications 4 ou 5 dans des conditions qui favorisent l'expression du polypeptide et ensuite, le polypeptide est obtenu.

**15.** Procédé de démucilagination d'huile végétale, **caractérisé en ce que**

a) éventuellement l'huile végétale à traiter est prétraitée,
b) est ajoutée à l'huile éventuellement ainsi prétraitée une solution aqueuse contenant un polypeptide selon l'une des revendications 7 à 11 ou une composition de phospholipase selon la revendication 12,
c) le mélange réactionnel selon le point b) est chauffé pendant 1 à 12 h à une température entre 30 °C et 70 °C, et
d) la phase aqueuse et la phase huileuse sont séparées l'une de l'autre.

Figur 1

Figur 2

Figur 3

```
  1   AGAGTCTGCC TATATTCTCT CTGAAAGGGT TGTCTTGAGT ATAGCTTCGG

 51   CATCATGGTC CAGTTCAAGT CTGTCCGTAC GCTGGCTGTC GCGGCGTTTG
            m   v   q   f   k   s   v   r   t   l   a   v   a   a   f

101   CTGCGCTGGG TGCTGCGGCG CCAGCAGGGT TGGCTGAGCG AGGTATGTCC
        a   a   l   g   a   a   a   p   a   g   l   a   e   r

151   GACGCTTCCT TAAGATTGGC TCTGGGTGGT GCTAACTACT AAGTAGATGT
                                                            d

201   GTCCGCGTCG GTGCTGCAAA AGTTGTCGTT GTTTGCGCAA TACTCTGCTG
        v   s   a   s   v   l   q   k   l   s   l   f   a   q   y   s   a

251   CCGCTTATTG TACCAACAAC ATCAATTCCA CGGGCACCAA GCTGACGTGC
        a   a   y   c   t   n   n   i   n   s   t   g   t   k   l   t   c

301   TCTGCTGGAA ACTGTCCTCT GGTCGAGGCA GCCAACACCA AGACCCTTGC
          s   a   g   n   c   p   l   v   e   a   a   n   t   k   t   l

351   GGAGTTCTAC GAGTAGGTCG ATCCCATGCA TGAGTAGCTC GCATATCTAA
        a   e   f   y   e

401   CAGAGCTGGT AGTTCCGAAT CGTTTGGAGA CACGGCAGGC TTCTTGGTTG
                  a   g   s   s   e   s   f   g   d   t   a   g   f   l   v

451   CAGACACCAC AAACAAGCTA CTCGTGGTCT CTTTCAGAGG AAGCCGCACG
        a   d   t   n   k   l   l   v   v   s   f   r   g   s   r   t

501   ATAGACAACT GGATTGCGAA TCTGGACTTT GTTCTGGACA GTGTCAGTGA
          i   d   n   w   i   a   n   l   d   f   v   l   d   s   v   s

551   TATTTGCAGC GGATGCGCCG CACACGGGGG CTTCTGGAAG TCCTGGGAAG
        d   i   c   s   g   c   a   a   h   g   g   f   w   k   s   w   e

601   TTGTTGCCAA TTCGCTGACG ACCGAGCTCA ACTCTGCGGT TAACACTTAC
          v   v   a   n   s   l   t   t   e   l   n   s   a   v   n   t   y

651   CCTGGCTATA CCATTGTCTT CACTGGACAT AGCCTCGGCG CTGCTCTTGC
          p   g   y   t   i   v   f   t   g   h   s   l   g   a   a   l

701   AACACTGGGG GCTACTACGC TGCGGAAAGC AGGGATTCCC ATTCAGCTGG
        a   t   l   g   a   t   t   l   r   k   a   g   i   p   i   q   l

751   TAAGTCATCC CTTGTCAACT TATGCAAGGG CGCAATGGGA CTAATTGATT

801   GTGAAGTATA ATTACGGATC CCCCCGTGTT GGAAACACGG CCTTGGCAAC
                    y   n   y   g   s   p   r   v   g   n   t   a   l   a

851   ATACATCACC GCACAGGGTC CCAATTACCG TGTCACACAC ACAAACGATA
        t   y   i   t   a   q   g   p   n   y   r   v   t   h   t   n   d

901   TTGTGCCCAG ACTCCCGCCC CAAGCTTTTG GCTTCAGCCA CCTTAGCCCG
        i   v   p   r   l   p   p   q   a   f   g   f   s   h   l   s   p
```

```
 951   GAGTACTGGA  TCACGAGCGG  CGACAACGTG  CCTGTCACGA  CGTCTGATAT
         e   y   w    i   t   s    g   d   n   v    p   v   t    t   s   d
1001   CACGGTCATC  CAGGGAATCG  ACTCAGACGC  TGGAAATTCG  GGAGAGGATA
         i   t   v   i    q   g   i    d   s   d    a   g   n   s    g   e   d
1051   TCACCAGCAT  CGAGGCCCAT  AATTGGTATC  TCGGCGATAT  TGATGCTTGT
         i   t   s    i   e   a   h    n   w   y    l   g   d    i   d   a   c
1101   CAATGAGACT  ATAAGCGGAG  TATATAACAG  CTTTGGATAG  TATAAAAGG
         q   –
1150   GCCAGTACAC  TTGGGCTAAC  GCATGAGGAA  TGACATTGAT  GACCTATCTT
1200   GCCAATGCAA  TCAGTTTTAT  AAGGAGAGTC  CTCATGATTG  ATTATGTCAA
1250   TTGGTATGGA  GTAGAAATAA  ACTGTACAGA  TCTCTGGATC  CGCCGAGTGG
1300   ACATTCATTA  TGAGGTTCTG  GGGAAGTTTG  TTTGGTTTGG  ACTTTGACAC
1350   CTGGAGTTTA  TCCCCATCTC  CATCAACTCG  TCTGATTGTG  GCTCGACGAG
1400   CGCATTCTTA  CTGAATGCTC  ATCTGTTTGA  ATAGAATATG  ATTAACGAGC
1450   AGTAACTCCC  ATTCCTTTCG  AACGCCTTTG  CGCAATTGAA  TCCATCCTTC
1500   CAACCCGTGC  AACTTCAACC  AGCCGCCCGG  GCGACTCTGC  GCATTCTCAA
1550   CATCTCTCGA  CCCGCCGCGA  TGGTCGCTGC  TCCATGCTGC  TGATACTCTT
1600   CTGTTATCAG  TAATCACGGA  AATTGTCATA
```

Figur 4

```
   1  AGAGTCTGCC TATATTCTCT CTGAAAGGGT TGTCTTGAGT ATAGCTTCGG
  51  CATCATGGTC CAGTTCAAGT CTGTCCGTAC GCTGGCTGTC GCGGCGTTTG
 101  CTGCGCTGGG TGCTGCGGCG CCAGCAGGGT TGGCTGAGCG AGGTATGTCC
 151  GACGCTTCCT TAAGATTGGC TCTGGGTGGT GCTAACTACT AAGTAGATGT
 201  GTCCGCGTCG GTGCTGCAAA AGTTGTCGTT GTTTGCGCAA TACTCTGCTG
 251  CCGCTTATTG TACCAACAAC ATCAATTCCA CGGGCACCAA GCTGACGTGC
 301  TCTGCTGGAA ACTGTCCTCT GGTCGAGGCA GCCAACACCA AGACCCTTGC
 351  GGAGTTCTAC GAGTAGGTCG ATCCCATGCA TGAGTAGCTC GCATATCTAA
 401  CAGAGCTGGT AGTTCCGAAT CGTTTGGAGA CACGGCAGGC TTCTTGGTTG
 451  CAGACACCAC AAACAAGCTA CTCGTGGTCT CTTTCAGAGG AAGCCGCACG
 501  ATAGACAACT GGATTGCGAA TCTGGACTTT GTTCTGGACA GTGTCAGTGA
 551  TATTTGCAGC GGATGCGCCG CACACGGGGG CTTCTGGAAG TCCTGGGAAG
 601  TTGTTGCCAA TTCGCTGACG ACCGAGCTCA ACTCTGCGGT TAACACTTAC
 651  CCTGGCTATA CCATTGTCTT CACTGGACAT AGCCTCGGCG CTGCTCTTGC
 701  AACACTGGGG GCTACTACGC TGCGGAAAGC AGGGATTCCC ATTCAGCTGG
 751  TAAGTCATCC CTTGTCAACT TATGCAAGGG CGCAATGGGA CTAATTGATT
 801  GTGAAGTATA ATTACGGATC CCCCCGTGTT GGAAACACGG CCTTGGCAAC
 851  ATACATCACC GCACAGGGTC CCAATTACCG TGTCACACAC ACAAACGATA
 901  TTGTGCCCAG ACTCCCGCCC CAAGCTTTTG GCTTCAGCCA CCTTAGCCCG
 951  GAGTACTGGA TCACGAGCGG CGACAACGTG CCTGTCACGA CGTCTGATAT
1001  CACGGTCATC CAGGGAATCG ACTCAGACGC TGGAAATTCG GGAGAGGATA
1051  TCACCAGCAT CGAGGCCCAT AATTGGTATC TCGGCGATAT TGATGCTTGT
1101  CAATGAGACT ATAAGCGGAG TATATAACAG CTTTGGATAG TATAAAAGG
1150  GCCAGTACAC TTGGGCTAAC GCATGAGGAA TGACATTGAT GACCTATCTT
1200  GCCAATGCAA TCAGTTTTAT AAGGAGAGTC CTCATGATTG ATTATGTCAA
1250  TTGGTATGGA GTAGAAATAA ACTGTACAGA TCTCTGGATC CGCCGAGTGG
1300  ACATTCATTA TGAGGTTCTG GGGAAGTTTG TTTGGTTTGG ACTTTGACAC
1350  CTGGAGTTTA TCCCCATCTC CATCAACTCG TCTGATTGTG GCTCGACGAG
1400  CGCATTCTTA CTGAATGCTC ATCTGTTTGA ATAGAATATG ATTAACGAGC
1450  AGTAACTCCC ATTCCTTTCG AACGCCTTTG CGCAATTGAA TCCATCCTTC
1500  CAACCCGTGC AACTTCAACC AGCCGCCCGG GCGACTCTGC GCATTCTCAA
1550  CATCTCTCGA CCCGCCGCGA TGGTCGCTGC TCCATGCTGC TGATACTCTT
1600  CTGTTATCAG TAATCACGGA AATTGTCATA
```

Figur 5

```
  1  MVQFKSVRTL  AVAAFAALGA  AAPAGLAERD  VSASVLQKLS  LFAQYSAAAY
 51  CTNNINSTGT  KLTCSAGNCP  LVEAANTKTL  AEFYEAGSSE  SFGDTAGFLV
101  ADTTNKLLVV  SFRGSRTIDN  WIANLDFVLD  SVSDICSGCA  AHGGFWKSWE
151  VVANSLTTEL  NSAVNTYPGY  TIVFTGHSLG  AALATLGATT  LRKAGIPIQL
201  YNYGSPRVGN  TALATYITAQ  GPNYRVTHTN  DIVPRLPPQA  FGFSHLSPEY
251  WITSGDNVPV  TTSDITVIQG  IDSDAGNSGE  DITSIEAHNW  YLGDIDACQ
```

Figur 6

Figur 7

Figur 8

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 0513709 A
- US 2007134777 A
- EP 0904357 A
- WO 2008040466 A
- EP 1788080 A
- WO 2008094847 A
- WO 9831790 A
- WO 2008040465 A
- WO 9845453 A
- EP 0808903 A
- WO 03097825 A
- WO 9500636 A
- EP 0449376 A
- WO 9428117 A
- DE 102009051013

### In der Beschreibung aufgeführte Nicht-Patentliteratur

- **ANNIKKA MUSTRANTA ; PIRKKO FORSSELL ; KAISA POUTANEN.** Comparison of Lipases and Phospholipases in the Hydrolysis of Phospholipids. *Process Biochemistry,* 01. Januar 1995, vol. 30 (5), 393-401
- **BIRCH et al.** Comparison of extracellular phospholipase activities in clinical and environmental Aspergillus fumigatus isolates. *Med Mycol,* 2004, vol. 42 (1), 81-86
- **REMENTERIA et al.** Genes and molecules involved in Aspergillus fumigatus virulence. *Rev Iberoam Micol,* 2005, vol. 22 (1), 1-23
- **NIERMAN et al.** Genomic sequence of the pathogenic and allergenic filamentous fungus Aspergillus fumigatus. *Nature,* 2005, vol. 438 (7071), 1151-6
- **SHEN et al.** Characterisation and expression of phospholipase B from the opportunistic fungus Aspergillus fumigatus. *FEMS Microbol Lett,* 2004, vol. 239 (1), 87-93
- **FEDOROVA et al.** Genomic Islands in the Pathogenic Filamentous Fungus Aspergillus fumigatus. *PloS Genet.,* 2008, vol. 4, e1000046
- **HIRSCHBERG, D.S.** A linear space algorithm for computing maximal common subsequences. *Commun Assoc Comput Mach,* 1975, vol. 18, 341-343
- **MYERS, E.W. ; W. MILLER.** Optimal alignments in linear space. *CABIOS,* 1988, vol. 4 (1), 11-17
- **CHAO, K-M ; W.R. PEARSON ; W. MILLER.** Aligning two sequences within a specified diagonal band. *CABIOS,* 1992, vol. 8 (5), 481-487
- **TOMIC et al.** *NAR,* 1990, vol. 18, 1656
- **GIEBEL ; SPRTIZ.** *NAR,* 1990, vol. 18, 4947
- **DAYHOFF et al.** Atlas of Protein Sequence and Structure, Natl. Biomed. Res. Found. 1978
- **SAMBROOK et al.** Molecular Cloning: A Laboratory Manual. Cold Spring Harbor Laboratory Press, 1989
- **PENTTILÄ et al.** *Gene,* 1987, vol. 61, 155-164
- **KOUKER ; JAEGER.** *Applied Environ. Microbiol.,* 1987, vol. 59, 211-213
- **WINKLER ; STUCKMANN.** *J. Bac.,* 1979, vol. 138, 663-670
- **HYNES, M.J et al.** *Mol. Cell. Biol.,* 1983, vol. 3, 1430-1439
- **NAKAI ; KANEHISA.** *Genomics,* 1992, vol. 14, 897-911

SEQUENZPROTOKOLL

<110> AB ENZYMES GMBH

<120> KLONIERUNG, EXPRESSION UND VERWENDUNG SAURER PHOSPHOLIPASEN

<130> 20581WO

<150> DE 10 2009 051 013.3
<151> 2009-10-28

<160> 5

<170> PatentIn version 3.3

<210> 1
<211> 1629
<212> DNA
<213> Aspergillus fumigatus

<220>
<221> CDS

<222> (55)..(142)

<220>
<221> CDS
<222> (197)..(362)

<220>
<221> CDS
<222> (404)..(749)

<220>
<221> CDS
<222> (807)..(1103)

<400> 1

```
agagtctgcc tatattctct ctgaaagggt tgtcttgagt atagcttcgg catc atg      57
                                                               Met
                                                               1

gtc cag ttc aag tct gtc cgt acg ctg gct gtc gcg gcg ttt gct gcg     105
Val Gln Phe Lys Ser Val Arg Thr Leu Ala Val Ala Ala Phe Ala Ala
            5                   10                  15

ctg ggt gct gcg gcg cca gca ggg ttg gct gag cga g gtatgtccga         152
Leu Gly Ala Ala Ala Pro Ala Gly Leu Ala Glu Arg
        20                  25

cgcttcctta agattggctc tgggtggtgc taactactaa gtag at  gtg tcc gcg    207
                                                    Asp Val Ser Ala

tcg gtg ctg caa aag ttg tcg ttg ttt gcg caa tac tct gct gcc gct     255
Ser Val Leu Gln Lys Leu Ser Leu Phe Ala Gln Tyr Ser Ala Ala Ala
    35                  40                  45

tat tgt acc aac aac atc aat tcc acg ggc acc aag ctg acg tgc tct     303
Tyr Cys Thr Asn Asn Ile Asn Ser Thr Gly Thr Lys Leu Thr Cys Ser
50                  55                  60                  65

gct gga aac tgt cct ctg gtc gag gca gcc aac acc aag acc ctt gcg     351
Ala Gly Asn Cys Pro Leu Val Glu Ala Ala Asn Thr Lys Thr Leu Ala
                70                  75                  80
```

```
gag ttc tac ga   gtaggtcgat cccatgcatg agtagctcgc atatctaaca g a         404
Glu Phe Tyr Glu


gct ggt agt tcc gaa tcg ttt gga gac acg gca ggc ttc ttg gtt gca         452
Ala Gly Ser Ser Glu Ser Phe Gly Asp Thr Ala Gly Phe Leu Val Ala
                90              95              100


gac acc aca aac aag cta ctc gtg gtc tct ttc aga gga agc cgc acg         500
Asp Thr Thr Asn Lys Leu Leu Val Val Ser Phe Arg Gly Ser Arg Thr
                105             110             115


ata gac aac tgg att gcg aat ctg gac ttt gtt ctg gac agt gtc agt         548
Ile Asp Asn Trp Ile Ala Asn Leu Asp Phe Val Leu Asp Ser Val Ser
            120             125             130


gat att tgc agc gga tgc gcc gca cac ggg ggc ttc tgg aag tcc tgg         596
Asp Ile Cys Ser Gly Cys Ala Ala His Gly Gly Phe Trp Lys Ser Trp
        135             140             145


gaa gtt gtt gcc aat tcg ctg acg acc gag ctc aac tct gcg gtt aac         644
Glu Val Val Ala Asn Ser Leu Thr Thr Glu Leu Asn Ser Ala Val Asn
150             155             160             165


act tac cct ggc tat acc att gtc ttc act gga cat agc ctc ggc gct         692
Thr Tyr Pro Gly Tyr Thr Ile Val Phe Thr Gly His Ser Leu Gly Ala
                170             175             180


gct ctt gca aca ctg ggg gct act acg ctg cgg aaa gca ggg att ccc         740
Ala Leu Ala Thr Leu Gly Ala Thr Thr Leu Arg Lys Ala Gly Ile Pro
            185             190             195


att cag ctg gtaagtcatc ccttgtcaac ttatgcaagg gcgcaatggg              789
Ile Gln Leu
            200


actaattgat tgtgaag tat aat tac gga tcc ccc cgt gtt gga aac acg         839
                    Tyr Asn Tyr Gly Ser Pro Arg Val Gly Asn Thr
                                    205             210


gcc ttg gca aca tac atc acc gca cag ggt ccc aat tac cgt gtc aca         887
Ala Leu Ala Thr Tyr Ile Thr Ala Gln Gly Pro Asn Tyr Arg Val Thr
            215             220             225


cac aca aac gat att gtg ccc aga ctc ccg ccc caa gct ttt ggc ttc         935
His Thr Asn Asp Ile Val Pro Arg Leu Pro Pro Gln Ala Phe Gly Phe
            230             235             240


agc cac ctt agc ccg gag tac tgg atc acg agc ggc gac aac gtg cct         983
Ser His Leu Ser Pro Glu Tyr Trp Ile Thr Ser Gly Asp Asn Val Pro
        245             250             255


gtc acg acg tct gat atc acg gtc atc cag gga atc gac tca gac gct        1031
Val Thr Thr Ser Asp Ile Thr Val Ile Gln Gly Ile Asp Ser Asp Ala
260             265             270             275


gga aat tcg gga gag gat atc acc agc atc gag gcc cat aat tgg tat        1079
Gly Asn Ser Gly Glu Asp Ile Thr Ser Ile Glu Ala His Asn Trp Tyr
            280             285             290


ctc ggc gat att gat gct tgt caa tgagactata agcggagtat ataacagctt       1133
Leu Gly Asp Ile Asp Ala Cys Gln
            295
```

```
tggatagtat aaaagggcca gtacacttgg gctaacgcat gaggaatgac attgatgacc    1193

tatcttgcca atgcaatcag ttttataagg agagtcctca tgattgatta tgtcaattgg    1253

tatggagtag aaataaactg tacagatctc tggatccgcc gagtggacat tcattatgag    1313

gttctgggga agtttgtttg gtttggactt tgacacctgg agtttatccc catctccatc    1373

aactcgtctg attgtggctc gacgagcgca ttcttactga atgctcatct gtttgaatag    1433

aatatgatta acgagcagta actcccattc ctttcgaacg cctttgcgca attgaatcca    1493

tccttccaac ccgtgcaact tcaaccagcc gcccgggcga ctctgcgcat tctcaacatc    1553

tctcgacccg ccgcgatggt cgctgctcca tgctgctgat actcttctgt tatcagtaat    1613

cacggaaatt gtcata                                                    1629
```

<210> 2
<211> 299
<212> PRT
<213> Aspergillus fumigatus

<400> 2

Met Val Gln Phe Lys Ser Val Arg Thr Leu Ala Val Ala Ala Phe Ala
1               5                   10                  15

Ala Leu Gly Ala Ala Ala Pro Ala Gly Leu Ala Glu Arg Asp Val Ser
            20                  25                  30

Ala Ser Val Leu Gln Lys Leu Ser Leu Phe Ala Gln Tyr Ser Ala Ala
            35                  40                  45

Ala Tyr Cys Thr Asn Asn Ile Asn Ser Thr Gly Thr Lys Leu Thr Cys
        50              55                  60

Ser Ala Gly Asn Cys Pro Leu Val Glu Ala Ala Asn Thr Lys Thr Leu
65              70                  75                  80

Ala Glu Phe Tyr Glu Ala Gly Ser Ser Glu Ser Phe Gly Asp Thr Ala
                85                  90                  95

Gly Phe Leu Val Ala Asp Thr Thr Asn Lys Leu Leu Val Val Ser Phe
            100                 105                 110

Arg Gly Ser Arg Thr Ile Asp Asn Trp Ile Ala Asn Leu Asp Phe Val
            115                 120                 125

Leu Asp Ser Val Ser Asp Ile Cys Ser Gly Cys Ala Ala His Gly Gly
    130                 135                 140

Phe Trp Lys Ser Trp Glu Val Val Ala Asn Ser Leu Thr Thr Glu Leu
145             150                 155                 160

```
        Asn Ser Ala Val Asn Thr Tyr Pro Gly Tyr Thr Ile Val Phe Thr Gly
                        165             170             175


        His Ser Leu Gly Ala Ala Leu Ala Thr Leu Gly Ala Thr Thr Leu Arg
                    180             185             190


        Lys Ala Gly Ile Pro Ile Gln Leu Tyr Asn Tyr Gly Ser Pro Arg Val
                    195             200             205


        Gly Asn Thr Ala Leu Ala Thr Tyr Ile Thr Ala Gln Gly Pro Asn Tyr
                210             215             220


        Arg Val Thr His Thr Asn Asp Ile Val Pro Arg Leu Pro Pro Gln Ala
        225             230             235             240


        Phe Gly Phe Ser His Leu Ser Pro Glu Tyr Trp Ile Thr Ser Gly Asp
                        245             250             255


        Asn Val Pro Val Thr Thr Ser Asp Ile Thr Val Ile Gln Gly Ile Asp
                    260             265             270


        Ser Asp Ala Gly Asn Ser Gly Glu Asp Ile Thr Ser Ile Glu Ala His
                275             280             285


        Asn Trp Tyr Leu Gly Asp Ile Asp Ala Cys Gln
                290             295
```

<210> 3
<211> 16
<212> PRT
<213> Aspergillus fumigatus

<400> 3

```
        Asp Val Ser Ala Ser Val Leu Gln Lys Leu Ser Leu Phe Ala Gln Tyr
        1               5               10              15
```

<210> 4
<211> 28
<212> DNA
<213> Artificial

<220>
<223> Primer

<400> 4
agagtctgcc tatattctct ctgaaagg          28

<210> 5
<211> 23
<212> DNA
<213> Artificial

<220>
<223> Primer

<400> 5
tatgacaatt tccgtgatta ctg        23